# EUROPEAN PATENT SPECIFICATION

(11) **EP 2 673 007 B1**
(45) Date of publication and mention of the grant of the patent: **10.02.2016**
(21) Application number: 12704635.7
(22) Date of filing: 10.02.2012
(51) Int. Cl.: A61L 2/03, A61L 2/22

(54) **ELECTROSTATIC DISINFECTANT TOOL**
ELEKTROSTATISCHES DESINFEKTIONSWERKZEUG
OUTIL DÉSINFECTANT ÉLECTROSTATIQUE

(30) Priority: 11.02.2011 US 201161442152 P; 28.07.2011 US 201161512834 P; 27.01.2012 US 201213360623
(43) Date of publication of application: 18.12.2013
(60) Divisional application: 15151932.9
(73) Proprietor: Finishing Brands Holdings Inc., Minneapolis, MN 55413 (US)
(72) Inventor: MICHELI, Paul R., Glenview, Illinois 60026 (US); MYERS, Steven Andrew, Glenview, Illinois 60026 (US); ARMSTRONG, Susan, Glenview, Illinois 60026 (US); METZGER, Gary Phillip, Glenview, Illinois 60026 (US); HASSELSCHWERT, Daniel J., Glenview, Illinois 60026 (US); GAJJAR, Nekheel S., Glenview, Illinois 60026 (US)
(74) Representative: Trinks, Ole
(86) International application number: PCT/US2012/024679
(87) International publication number: WO 2012/109556

(56) References cited:
- EP-A2- 0 853 980
- WO-A1-00/67805
- WO-A1-2004/101165
- US-A- 4 971 257
- US-A- 5 218 305
- US-A1- 2003 143 109
- US-A1- 2010 147 700

## Description

### BACKGROUND

The present disclosure relates generally to disinfection devices for use in various industries, such as healthcare and the food industry.

A system according to the preamble of claim 1 is known from US 2010/147 700 A1. A liquid is dispensed from an apparatus to a surface or volume so as to create an electrically conductive path by the treated liquid by generating an alternating electric field from the apparatus to the surface or volume. The electric field is sufficient to destroy microorganisms from the surface or volume.

From WO 00/678 05 A1 an apparatus for cleaning particulate matter and chemical contaminants from hands is known. It comprises a mechanical-cleaning device having a pressurized gas source directing a flow of pressurized ionized gas into the hand-cleaning volume, and a chemical-cleaning device having an activating nebulizer emitting an activated cleaning mist into the hand-cleaning volume. US 2003/143 109 A1 discloses methods for sterilizing or disinfecting a surface, which comprise applying an electrostatically charged aerosol of a disinfecting solution onto said surface and allowing said aerosol to remain in contact with said surface for a time sufficient to achieve the desired degree of sterilization. US 4 971 257 A describes an electrostatic particle spraying apparatus comprising a hand-held triggering mechanism provided with a self-contained source of high D.C. voltage and a coupling sleeve for a conventional aerosol can. Squeezing a trigger mechanism creates an electric potential in an electrode positioned in the projected path of particle spray from the aerosol can. A further electrostatic spraying device is known from EP 0 853 980 A2 and US 5 218 305 A showing an electrostatic spray gun having a self-contained power supply including a high voltage electrostatic voltage and current source.

Disinfectants are used by individuals, schools, businesses, government institutions, and various industries every day. Many industries, such as healthcare and the food industry, use sanitation and disinfection processes to clean work surfaces, tools, instruments, and other materials. As appreciated, the quantity of these disinfectants can be quite large, which can result in significant expenses, environmental impact, and potential exposure of the disinfectants to individuals. Furthermore, current sanitation and disinfection methods are frequently ineffective, slow, and labor intensive. For example, sanitation and disinfection agents, such as disinfectants and various chemicals, may be overused and/or used ineffectively leading to increased costs and environmental impact. There are also environmental concerns associated with the disposal of used disinfectants and chemicals. Furthermore, the manual application of these agents can be time consuming, inconsistent, and unsuitable for cleaning areas that are hidden or difficult to access. For example, a mop, brush, or cloth may be unable to reach corners, recesses, and other areas, thereby resulting in incomplete sanitation or disinfection. Similarly, mops, brushes, and cloths may be reused, potentially causing bacteria to spread to other surfaces.

Accordingly, a need exists to improve on existing disinfection techniques. The disclosed techniques provide an effective disinfectant system, which increases protection of the environment and individuals. Additionally, the disclosed techniques may be used with existing disinfectants to improve their effectiveness and potentially reduce their required concentrations, resulting in a safer disinfecting process and a lower impact on the environment.

### SUMMARY

In an embodiment, a system comprises an electrostatic tool configured to output a discharge to kill a biological cell by electroporation, wherein the electrostatic tool comprises: an electrostatic applicator configured to output an electrostatic field, wherein the discharge comprises the electrostatic field. The electrostatic tool further comprises a gas passage con-figured to flow a gas, the electrostatic applicator being configured to ionize the gas to produce an ionized gas, and the discharge comprises the electrostatic field and the ionized gas.

These and other features, aspects, and advantages of the present disclosure will become better understood when the following detailed description is read with reference to the accompanying drawings in which like characters represent like parts throughout the drawings.

### BRIEF DESCRIPTION OF THE DRAWINGS

FIG. 1 is a block diagram illustrating an electrostatic disinfectant tool having an electrostatic applicator, wherein the electrostatic disinfectant tool is configured to output a discharge to kill a biological cell.
FIG. 2 is a block diagram illustrating an electrostatic disinfectant tool having a gas assisted electrostatic applicator, wherein the electrostatic disinfectant tool is configured to output a discharge to kill a biological cell.
FIG. 3 is a block diagram illustrating an electrostatic disinfectant tool having a spray assisted electrostatic applicator, wherein the electrostatic disinfectant tool is configured to output a discharge to kill a biological cell.
FIG. 4 is a flow chart illustrating a process for applying a discharge including an electrostatic field to kill a biological cell.
FIG. 5 is a schematic flow diagram illustrating a process of applying a discharge including an electrostatic field to kill a biological cell.
FIG. 6 is a flow chart illustrating a process for applying a discharge including an electrostatic field and an ionized gas to kill a biological cell.
FIG. 7 is a schematic flow diagram illustrating a process of applying a discharge including an electrostatic field and an ionized gas to kill a biological cell.
FIG. 8 is a flow chart illustrating a process for applying a discharge including an electrostatic field, an ionized gas, and a charged spray to kill a biological cell.
FIG. 9 is a schematic flow diagram illustrating a process of applying a discharge including an electrostatic field, an ionized gas, and a charged spray to kill a biological cell.
FIG. 10 is a flow chart illustrating a process for applying a discharge including an electrostatic field, an ionized gas, and a charged biocide spray to kill a biological cell.
FIG. 11 is a schematic flow diagram illustrating a process of applying a discharge including an electrostatic field, an ionized gas, and a charged biocide spray to kill a biological cell.
FIG. 12 is a schematic of an embodiment of an electrostatic disinfectant tool having an electrostatic field diffuser, a gravity applicator, and a pressurized gas cartridge.
FIG. 13 is a schematic of an embodiment of an electrostatic disinfectant tool having an electrostatic diffuser, a gravity applicator, and a pressurized gas cartridge.
FIG. 14 is a partial front view of the electrostatic disinfectant tool, taken along line 14-14 of FIG. 13, illustrating an embodiment of the electrostatic diffuser.
FIG. 15 is a partial cross-sectional side view of the electrostatic disinfectant tool, taken within line 15-15 of FIG. 13, illustrating an embodiment of the electrostatic applicator and the electrostatic diffuser.
FIG. 16 is a partial cross-sectional side view of the electrostatic disinfectant tool, taken within line 15-15 of FIG. 13, illustrating an embodiment of the electrostatic applicator and the electrostatic diffuser.
FIG. 17 is a cross-sectional side view of an embodiment of a gravity applicator that may be used in conjunction with the electrostatic disinfectant tool.
FIG. 18 is a schematic of an embodiment of an electrostatic disinfectant tool having an electrostatic applicator and a gas driven turbine.
FIG. 19 is a perspective view of an embodiment of an electrostatic disinfectant tool having a disinfectant compartment with hand receptacles and an access door.
FIG. 20 is a top view of the electrostatic disinfectant tool of FIG. 19, illustrating internal components within the disinfectant compartment.
FIG. 21 is a cross-sectional side view of the electrostatic disinfectant tool of FIG. 19, illustrating internal components within the disinfectant compartment.
FIG. 22 is a schematic of an example embodiment of the electrostatic disinfectant tool system, where the electrostatic disinfectant tool system includes an electrostatic module coupled to a spray bottle.
FIG. 23 is a block diagram of an example embodiment of the electrostatic disinfectant tool, where the electrostatic disinfectant tool has the electrostatic module.
FIG. 24 is a schematic of an example embodiment of the electrostatic module, illustrating the electrostatic module coupled to the base of a spray bottle.
FIG. 25 is a schematic of an example embodiment of the electrostatic module, illustrating the electrostatic module coupled to the base of a spray bottle.
FIG. 26 is a partial perspective view an example embodiment of the spray bottle, which may be configured for use with the electrostatic module.
FIG. 27 is a schematic of an example embodiment of the electrostatic module, which may be coupled to the spray bottle shown in FIG. 26.

### DETAILED DESCRIPTION

One or more specific embodiments of the present disclosure will be described below. In an effort to provide a concise description of these embodiments, all features of an actual implementation may not be described in the specification.

Various embodiments of the present disclosure provide a tool for providing a discharge (e.g., spray) to disinfect, sanitize, and/or sterilize a target object. In certain embodiments, the tool may create an electrostatic field to improve coverage of a liquid spray (e.g., a biocide spray) on a target object, e.g., by inducing the spray to wrap around the target object and cover all sides of the target object with the biocide. Furthermore, the tool uses the charge from the electrostatic field to help kill undesirable cells in addition to the improved coverage (e.g., wrap-around) of the spray around the target object. As discussed in detail below, the discharge includes electrostatic field, an ionized gas, a charged liquid (e.g., a charged biocide), or a combination thereof, which effectively reduce or eliminate undesirable cells, such as bacteria, in various environments (e.g., environments in the healthcare industry or the food industry).

It should be appreciated that the term sterilization refers to the killing of all microorganisms in a material or on the surface of an object. For example, sterilization refers to killing all microorganisms, including but not limited to, transmissible agents such as fungi, bacteria, viruses, spore forms, and so forth. The term sanitization refers to the cleaning of pathogenic microorganisms, such as pathogenic microorganisms on food preparation equipment (e.g., in a kitchen), eating utensils, and other items used in the food industry. The term disinfection refers to reducing the number of viable microorganisms present on an object, but not necessarily killing all microorganisms on the object. The term disinfection is intended to be inclusive of sterilization and sanitization. The disclosed embodiments of the tool are intended to include sterilizer tools, sanitizer tools, disinfectant tools, or any combination thereof. Thus, any use of these terms in the following discussion is not intended to be limiting, but rather are intended to equally apply to disinfecting, sanitizing, and/or sterilizing.

Various embodiments of the present disclosure provide an electrostatic disinfectant tool that provides enhanced effectiveness of disinfecting surface areas, instruments, tools, and other materials. In certain embodiments, the electrostatic disinfectant tool includes an electrostatic applicator having an electrostatic diffuser, wherein the electrostatic disinfectant tool is configured to apply an electrostatic field to a surface or object to be disinfected. In particular, the electrostatic disinfectant tool may kill at least some or all biological cells (e.g., bacteria) by electroporation. As used herein, "electroporation" refers to the process of subjecting a biological cell to a high intensity electrostatic charge, which causes the cell membrane of the biological cell to porate or create one or more openings into the cell membrane.

In certain embodiments, the electrostatic disinfectant tool intentionally over-porates the cell membrane to cause the cell membrane to rupture (e.g., over-poration), thereby killing the biological cell. In some embodiments, the electrostatic disinfectant tool is assisted by a fluid output, such as a gas output or a liquid output (e.g., a liquid spray output). For example, the electrostatic disinfectant tool may porate the cell membrane, while also injecting a fluid (e.g., gas and/or liquid) through the cell opening into the cell membrane to assist with killing the biological cell. In various alternative embodiments, the fluid may include a biocide, an ionized gas, an electrostatically charged liquid (e.g., water or biocide), or any suitable combination of these.

In some embodiments, the electrostatic disinfectant tool includes a portable or stationary electrostatic disinfectant tool. For example, the electrostatic disinfectant tool may include a hand-held electrostatic disinfectant tool, such as a gun-shaped electrostatic disinfectant tool, or a portable or stationary unit with a disinfectant compartment, which may include hand receptacles and an access cover. The electrostatic disinfectant tool may be designed specifically for a particular industry, such as healthcare or the food industry. Thus, the electrostatic disinfectant tool may be integrated with other equipment in the target industry.

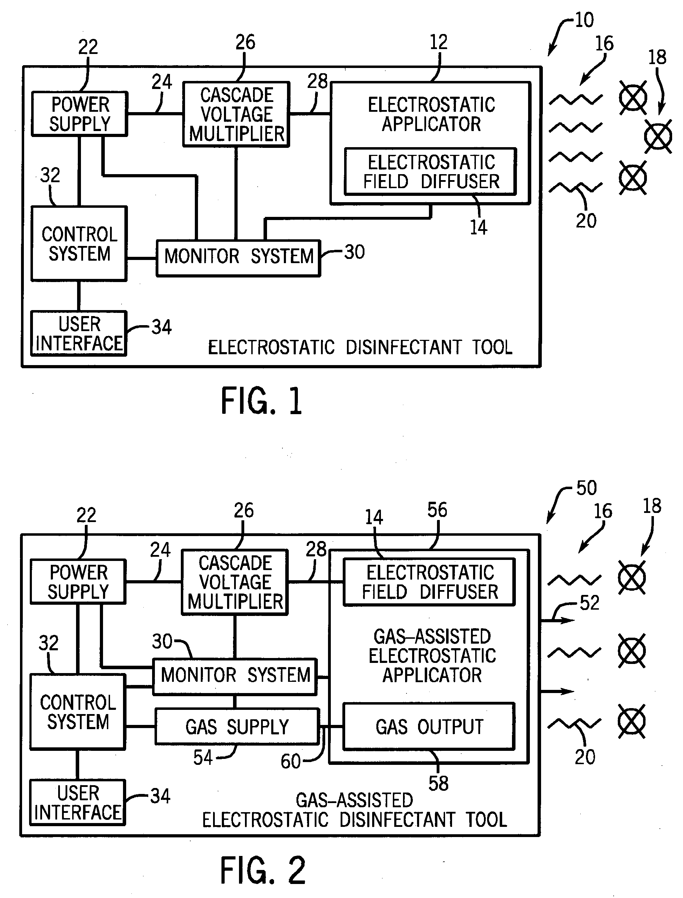

Referring now to FIG. 1, an example embodiment of an electrostatic disinfectant tool system 10 includes an electrostatic applicator 12 having an electrostatic field diffuser 14 is shown. The electrostatic disinfectant tool system 10 is configured to apply a discharge 16 to kill biological cells 18 by electroporation. In the illustrated example, the discharge 16 includes an electrostatic field 20. In certain embodiments, the discharge 16 may consist essentially of, or entirely of, the electrostatic field 20. However, as discussed further below, the discharge 16 may be supplemented or assisted with one or more fluids (e.g., gas or liquid).

As illustrated in FIG. 1, an electrostatic diffuser 14 receives an electrostatic potential and applies the electrostatic field 20 over the area to be disinfected. The electrostatic diffuser 14 may be configured to apply the discharge 16 over a wide area. In certain embodiments, the electrostatic diffuser 14 includes a wide surface or plate (e.g., a flat plate, a curved plate, or an angled plate) configured to distribute the discharge. In such embodiments, the plate may include an electrode grid having a plurality of protruding electrodes, e.g., 10 to 1000 or more electrodes. The electrodes may be configured to apply the discharge 16, such as an electrostatic charge, onto a surface or object to be disinfected. For example, the electrodes may exhibit a negative charge that is created by the combination of a low-voltage power supply and a cascade section that boosts the input voltage at the electrode tip. For example, the voltage at the electrode tip may be boosted to 85kV. The current flow may be very low, on the order of approximately 50-100 microamps, so that the charge is essentially a DC static charge. The opposite charge is created on the object which is to be disinfected.

In the illustrated example, the electrostatic disinfectant tool system 10 includes a power supply 22, which provides power 24 to a cascade voltage multiplier 26. The power supply 22 may include an external power source or an internal power source, such as an electrical generator. The cascade voltage multiplier 26 receives the power 24 from the power supply 22 and converts the power 24 to a higher voltage power 28 to be applied to the electrostatic field diffuser 14. More specifically, the cascade voltage multiplier 26 may apply power 28 with a voltage between approximately 55 kV and 85 kV or greater to the electrostatic field diffuser 14. For example, the power 28 may be at least approximately 55, 60, 65, 70, 75, 80, 85, 90, 95, 100, or greater kV. As will be appreciated, the cascade voltage multiplier 26 may include diodes and capacitors and also may be removable. In certain embodiments, the cascade voltage multiplier 26 may also include a switching circuit configured to switch the power 28 applied to the electrostatic field diffuser 14 between a positive and a negative voltage.

As shown in FIG. 1, the electrostatic disinfectant tool system 10 further includes a monitor system 30 and a control system 32, each of which may be powered by the power supply 22. The monitor system 30 may be coupled to the cascade voltage multiplier 26 and the electrostatic applicator 12 to monitor various operating parameters and conditions. For example, the monitor system 30 may be configured to monitor the voltage of the power 24 received by the cascade voltage multiplier 26 from the power supply 22. Similarly, the monitor system 30 may be configured to monitor the voltage of the power 28 output by the cascade voltage multiplier 26. Furthermore, the monitor system 30 may be configured to monitor the voltage of the electrostatic field 20 applied by the electrostatic field diffuser 14. The control system 32 may also be coupled to the monitor system 30. In certain embodiments, the control system 32 may be configured to allow a user to adjust various settings and operating parameters based on information collected by the monitor system 30. Specifically, the user may adjust settings or parameters with a user interface 34 coupled to the control system 32. For example, the control system 32 may be configured to allow a user to adjust the voltage of the electrostatic field 20 using a knob, dial, button, or menu on the user interface 34. The user interface 34 may further include an ON/OFF switch and a display for providing system feedback, such as voltage or current levels, to the user. In certain embodiments, the user interface 34 may include a touch screen to enable both user input and display of information relating to the electrostatic disinfectant tool system 10.

Referring now to FIG. 2, an example embodiment of a gas-assisted electrostatic disinfectant tool system 50 configured to apply a discharge 16 with both an electrostatic field 20 and an ionized gas 52 is shown. The illustrated gas-assisted electrostatic disinfectant tool system 50 includes elements and element numbers similar to the electrostatic disinfectant tool system 10 shown in FIG. 1. Additionally, the gas-assisted electrostatic disinfectant tool system 50 includes a gas supply 54 and a gas-assisted electrostatic applicator 56 having a gas output 58. The gas supply 54 provides a gas 60 to the gas output 58 of the gas-assisted electrostatic applicator 56. In certain embodiments, the gas supply 54 is an internal gas supply, such as a gas cartridge, a fan, or a compressor. For example, the gas supply 54 may be an internal fan or compressor, which is powered by an internal power supply 22, such as a battery or an electrical generator. In other embodiments, the gas supply 54 is an external gas supply, such as a pressurized gas tank, a fan, a compressor (e.g., an air compressor), or a combination thereof. Additionally, the gas supply 54 may include nitrogen, carbon dioxide, air, any other suitable gas, or a combination of these. The gas-assisted electrostatic applicator 56 is configured to ionize the gas 60 to produce the ionized gas 52. Thus, the ionized gas 52 may include ionized nitrogen, ionized carbon dioxide, ionized air, or a combination thereof.

As discussed in detail below, the electrostatic field diffuser 14 may include one or more electrodes, which apply the power 28 received from the cascade voltage multiplier 26 to the gas 60 to create the ionized gas 52. The ionized gas 52 is applied to a surface or object to be disinfected by the gas-assisted electrostatic applicator 56. In this manner, the discharge 16 kills biological cells with both the electrostatic field 20 and the ionized gas 52. For example, the electrostatic field 20 may porate or over-porate the cell membrane, while the ionized gas 52 supplements the poration by the electrostatic field 20. In particular, the ionized gas 52 penetrates the cell membrane by passing through openings in the cell wall caused by the electroporation, thereby breaking down the cell membrane.

As shown in FIG. 2, the gas supply 54 is coupled to the monitor system 30 and the control system 32. In certain embodiments, the monitor system 30 is configured to monitor various operating conditions and parameters of the gas supply 54. For example, the monitor system 32 may monitor the internal pressure of the gas supply 54 and the flow rate of the gas 60 from the gas supply 54 to the gas output 58 of the gas-assisted electrostatic applicator 56. Additionally, the control system 32 is configured to regulate one or more operating parameters of the gas supply 60 based on feedback received from the monitor system 30 or based on user input from the user interface 34. For example, the user interface 34 may include dials, knobs or buttons to allow a user to control the flow rate of the gas 60 from the gas supply 54 to the gas output 58 of the gas-assisted electrostatic applicator 56. Moreover, the user interface 34 may include a display (e.g., a touch screen) to communicate system feedback, such as the internal pressure of the gas supply 54, to a user.

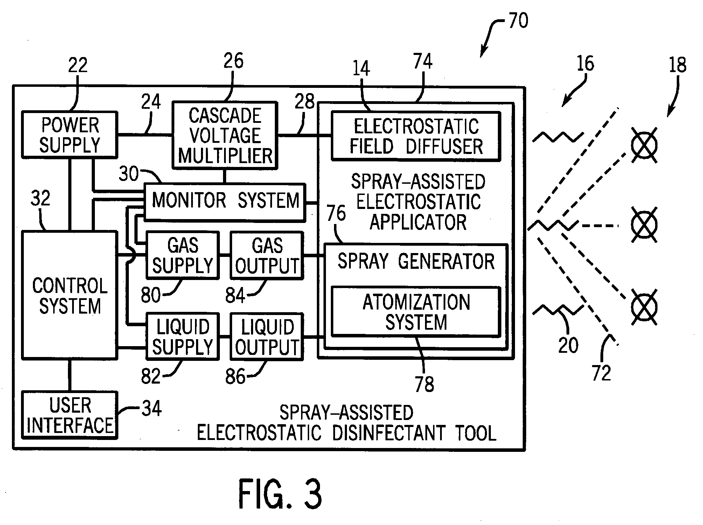

Referring now to FIG. 3, an example embodiment of a spray-assisted electrostatic disinfectant tool system 70 configured to apply a discharge 16 with an electrostatic field 20 and a charged liquid spray 72 (and optionally an ionized gas 52) is shown. The illustrated spray-assisted electrostatic disinfectant tool system 70 includes elements and element numbers similar to the electrostatic disinfectant tool system 10 provided in FIGS. 1 and 2. Additionally, the spray-assisted electrostatic disinfectant tool system 70 of FIG. 3 includes a spray-assisted electrostatic applicator 74 having a spray generator 76. The spray generator 76 includes an atomization system 78. As discussed below, the atomization system 78 of the spray generator 76 atomizes a liquid and produces a liquid spray. The spray-assisted electrostatic applicator 74 is configured to electrically charge the liquid spray to produce the charged liquid spray 72. For example, the electrostatic field diffuser 14 of the spray-assisted electrostatic applicator 74 may include one or more electrodes, which apply the power 28 received from the cascade voltage multiplier 26 to the liquid spray to create the charged liquid spray 72. In this manner, the discharge 16 kills biological cells with both the electrostatic field 20 and the charged liquid spray 72. For example, the electrostatic field 20 may porate or over-porate the cell membrane, while the charged liquid spray 72 supplements the poration by the electrostatic field 20. In particular, the charged liquid spray 72 penetrates the cell membrane by passing through openings in the cell wall caused by the electroporation, thereby breaking down the cell membrane. In certain embodiments, the discharge 16 may include the electrostatic field 20, the ionized gas 52, and the charged liquid spray 72 to enhance the effectiveness of the electrostatic disinfectant tool system 70.

As shown in FIG. 3, the spray-assisted electrostatic disinfectant tool system 70 includes a gas supply 80 and a liquid supply 82. The gas supply 80 provides a gas to the spray generator 76 through a gas output 84. Similarly, the liquid supply 82 provides a liquid to the spray generator 76 through a liquid output 86. In the illustrated example, the atomization system 78 is a gas atomization system, which uses the gas from the gas supply 80 to atomize the liquid from the liquid supply 82 to produce a liquid spray. For example, the atomization system 78 may apply gas jets toward a liquid stream, thereby breaking up the liquid stream into a liquid spray. In other embodiments, the atomization system 78 may include a rotary atomizer, an airless atomizer, or another suitable atomizer. In the illustrated example, the gas supply 80 is an internal or external gas supply, which may include, nitrogen, carbon dioxide, air, any other suitable gas, or a combination these. For example, the gas supply 80 may be a pressurized gas cartridge mounted directly on or within the electrostatic disinfection system 70, or the gas supply 80 may be a separate pressurized gas tank or gas compressor. In various alternative embodiments, the liquid supply 80 may include an internal or external liquid supply. For example, the liquid supply 80 may include a gravity applicator, a siphon cup, or a pressurized liquid tank. Further, the liquid supply 80 may be configured to hold or contain water, a biocide material, or any other suitable liquid.

As illustrated in FIG. 3, the monitor system 30 is coupled to and is configured to monitor, the spray-assisted electrostatic applicator 74. In addition to being configured to monitor the voltage of the electrostatic field 20 applied by the electrostatic field diffuser 14, as mentioned above, the monitor system 30 is configured to monitor the flow rate of the charged liquid spray 72 from the spray-assisted electrostatic applicator 74. Additionally, the monitor system 30 is configured to monitor the rate at which the spray generator 76 produces the liquid spray. The monitor system 30 is coupled to the gas supply 80 and the liquid supply 82. The monitor system 30 monitors the internal pressure of the gas supply 80 and the flow rate of gas from the gas supply 80 to the gas output 84. Similarly, the monitor system 30 monitors the internal pressure of the liquid supply 82 and the flow rate of liquid from the liquid supply 82 to the liquid output 86.

As shown, the gas supply 80 and the liquid supply 82 are coupled to the control system 32. As will be appreciated, the control system 32 may be configured to adjust one or more operating parameters of the gas supply 80 and the liquid supply 82. More particularly, the control system 32 may be configured to adjust one or more operating parameters of the gas supply 80 or the liquid supply 82 based on information received from the monitor system 30 or based on user input received from the user interface 34. For example, the control system 30 may control the flow rate of the gas from the gas supply 80 to the gas output 84 or the flow rate of the liquid from the liquid supply 82 to the liquid output 86. The user interface 34 may include knobs, dials, or buttons to allow a user to manually adjust the various operating parameters of the gas supply 80 and the liquid supply 82. The user interface 34 may include a display (e.g., a touch screen) to communicate system feedback, such as the internal pressure of the gas supply 80 and the liquid supply 82, to a user.

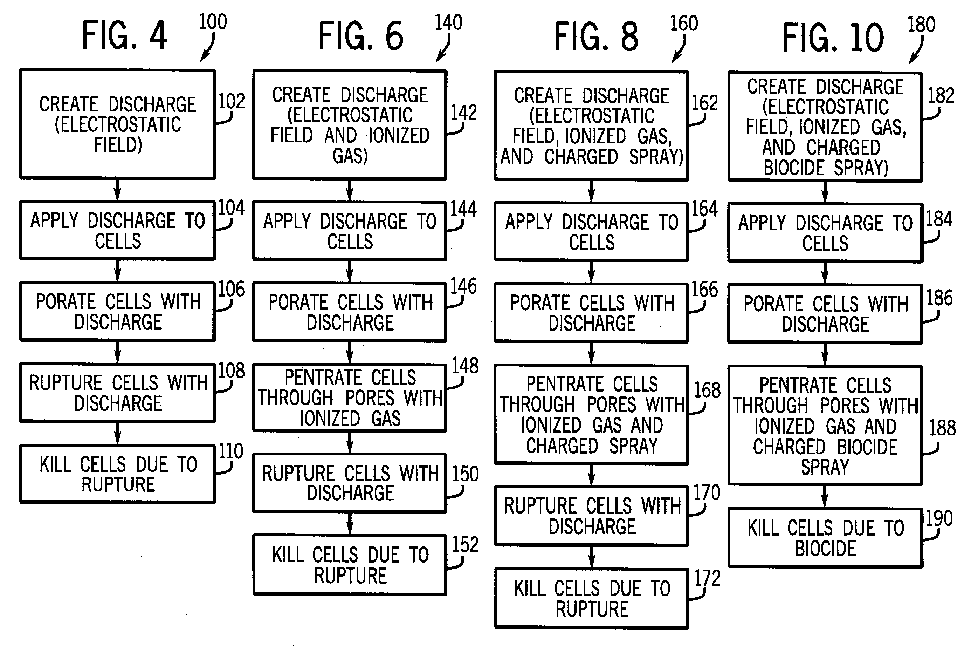

As illustrated in FIG. 4, an example method for applying a discharge 16 including an electrostatic field 20 to kill biological cells 18 operates according to sequence 100. A discharge 16 including an electrostatic field 20 is created or formed, as indicated by block 102. The discharge 16 is applied to the biological cells 18, as indicated by block 104. The electrostatic field 20 of the discharge 16 porates the biological cells 18, as indicated by block 106. The electrostatic field 20 of the discharge 16 further causes the biological cells 18 to over-porate, leading to the rupture of the cell membranes of the biological cells 18, as indicated by block 108. The biological cells 18 die due to rupture, as indicated by block 110.

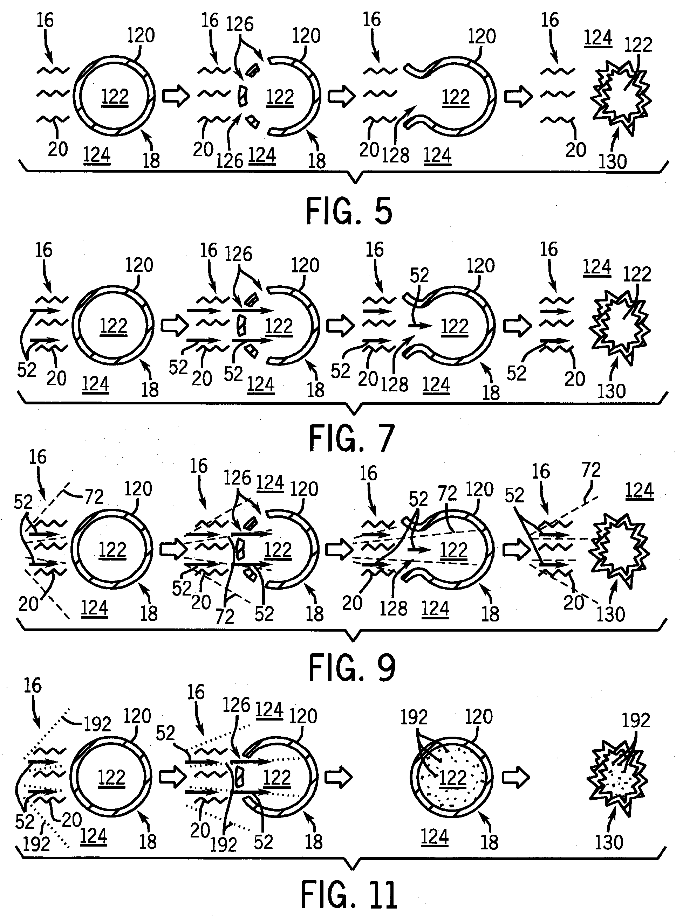

FIG. 5 is a schematic flow diagram illustrating the method of FIG. 4 of applying the discharge 16 having an electrostatic field 20 to kill a biological cell 18. As shown, after the discharge 16 having the electrostatic field 20 is created, the discharge 16 is applied to a biological cell 18. Specifically, the discharge 16 is applied to a cell membrane 120 of the biological cell 18, which surrounds an inner volume 122 of the biological cell 18. As will be appreciated, the cell membrane 120 protects the inner volume 122 of the cell from a surrounding environment 124. Thereafter, the electrostatic field 20 of the discharge 16 causes the cell membrane 120 of the biological cell 18 to porate. More particularly, pores 126 form in the cell membrane 120 of the biological cell 18. The pores 126 cause the inner volume 122 of the biological cell 18 to become accessible by the discharge 16. The pores 126 further expose the inner volume 122 of the biological cell 18 to the surrounding environment 124. Subsequently, the application of the electrostatic field 20 of the discharge 16 to the cell membrane 120 of the biological cell 18 causes over-poration of the cell membrane 120. The over-poration of the cell membrane 120 leads to rupture 128 of the cell membrane 120, leaving the inner volume 122 of the biological cell 18 exposed to the surrounding environment 124. The rupture 128 of the cell membrane 120 results in the biological cell 18 dying and becoming a dead biological cell 130.

As illustrated in FIG. 6, an example method for applying a discharge 16 including an electrostatic field 20 and an ionized gas 52 to kill biological cells 18 operates according to sequence 140. A discharge 16 including an electrostatic field 20 and an ionized gas 52 is created or formed, as indicated by block 142. The discharge 16 is applied to the biological cells 18, as indicated by block 144. The electrostatic field 20 and the ionized gas 52 of the discharge 16 porate the biological cells 18, as indicated by block 146. Pores created by the poration of the biological cells 18 allow the ionized gas 52 to penetrate the cells, as indicated by block 148. The electrostatic field 20 and the ionized gas 52 of the discharge 16 cause the biological cells 18 to over-porate, leading to the rupture of the cell membranes of the biological cells 18, as indicated by block 150. The biological cells 18 die due to rupture, as indicated by block 152.

FIG. 7 is a schematic flow diagram illustrating the method of FIG. 6 of applying the discharge 16 having an electrostatic field 20 and an ionized gas 52 to kill a biological cell 18. As shown, after the discharge 16 having the electrostatic field 20 and the ionized gas 52 is created, the discharge 16 is applied to a biological cell 18. Specifically, the discharge 16 is applied to a cell membrane 120 of the biological cell 18, which surrounds an inner volume 122 of the biological cell 18. As will be appreciated, the cell membrane 120 protects the inner volume 122 of the cell from a surrounding environment 124. Thereafter, the electrostatic field 20 and the ionized gas 52 of the discharge 16 cause the cell membrane 120 of the biological cell 18 to porate. More particularly, pores 126 form in the cell membrane 120 of the biological cell 18. The pores 126 cause the inner volume 122 of the biological cell 18 to become accessible by the discharge 16. More specifically, the ionized gas 52 penetrates the cell membrane 120 of the biological cell and enters the inner volume 122 of the biological cell, as shown. The pores 126 further expose the inner volume 122 of the biological cell 18 to the surrounding environment 124. Subsequently, the application of the electrostatic field 20 and the ionized gas 52 of the discharge 16 to the cell membrane 120 and the inner volume 122 of the biological cell 18 cause over-poration of the cell membrane 120. The over-poration of the cell membrane 120 leads to a rupture 128 of the cell membrane 120, leaving the inner volume 122 of the biological cell 18 exposed to the surrounding environment 124. The rupture 128 of the cell membrane 120 results in the biological cell 18 dying and becoming a dead biological cell 130.

As illustrated in FIG. 8, an example method for applying a discharge 16 including an electrostatic field 20, an ionized gas 52, and a charged liquid spray 72 to kill biological cells 18 operates according to sequence 160. A discharge 16 including an electrostatic field 20, an ionized gas 52, and a charged liquid spray 72 is created, as indicated by block 162. The discharge 16 is applied to the biological cells 18, as indicated by block 164. The electrostatic field 20, the ionized gas 52, and the charged liquid spray 72 of the discharge 16 porate the biological cells 18, as indicated by block 166. Pores created by the poration of the biological cells 18 allow the ionized gas 52 and the charged liquid spray 72 to penetrate the cells, as indicated by block 168. The electrostatic field 20, the ionized gas 52, and the charged liquid spray 72 of the discharge 16 cause the biological cells 18 to over-porate, leading to the rupture of the cell membranes of the biological cells 18, as indicated by block 170. The biological cells 18 die due to rupture, as indicated by block 172.

FIG. 9 is a schematic flow diagram illustrating the method of FIG. 8 of applying the discharge 16 having an electrostatic field 20, an ionized gas 52, and a charged liquid spray 72 to kill a biological cell 18. As shown, after the discharge 16 having the electrostatic field 20, the ionized gas 52, and the charged liquid spray 72 is created, the discharge 16 is applied to a biological cell 18. Specifically, the discharge 16 is applied to a cell membrane 120 of the biological cell 18, which surrounds an inner volume 122 of the biological cell 18. As will be appreciated, the cell membrane 120 protects the inner volume 122 of the cell from a surrounding environment 124. Thereafter, the electrostatic field 20, the ionized gas 52, and the charged liquid spray 72 of the discharge 16 cause the cell membrane 120 of the biological cell 18 to porate. More particularly, pores 126 form in the cell membrane 120 of the biological cell 18. The pores 126 cause the inner volume 122 of the biological cell 18 to become accessible by the discharge 16. More specifically, the ionized gas 52 and the charged liquid spray 72 penetrates the cell membrane 120 of the biological cell and enter the inner volume 122 of the biological cell, as shown. The pores 126 expose the inner volume 122 of the biological cell 18 to the surrounding environment 124. Subsequently, the application of the electrostatic field 20, the ionized gas 52, and the charged liquid spray 72 of the discharge 16 to the cell membrane 120 and the inner volume 122 of the biological cell 18 cause over-poration of the cell membrane 120. The over-poration of the cell membrane 120 leads to a rupture 128 of the cell membrane 120, leaving the inner volume 122 of the biological cell 18 exposed to the surrounding environment 124. The rupture 128 of the cell membrane 120 results in the biological cell 18 dying and becoming a dead biological cell 130.

As illustrated in FIG. 10, an example method for applying a discharge 16 including an electrostatic field 20, an ionized gas 52, and a charged biocide spray to kill biological cells 18 operates according to sequence 180. A discharge 16 including an electrostatic field 20, an ionized gas 52, and a charged biocide spray is created, as indicated by block 182. The discharge 16 is applied to the biological cells 18, as indicated by block 184. The electrostatic field 20, the ionized gas 52, and the charged biocide spray of the discharge 16 porate the biological cells 18, as indicated by block 186. Pores created by the poration of the biological cells 18 allow the ionized gas 52 and the charged biocide spray to penetrate the cells, as indicated by block 188. In certain embodiments, the electrostatic field 20, the ionized gas 52 and the charged biocide spray cause the cell membranes 120 of the biological cells 18 to rupture and kill the cells 18. In other embodiments, the electrostatic field 20, the ionized gas 52 and the charged biocide spray porate the cell membranes 120 of the biological cells 18, while the biocide effectively kills the biological cells 18 from the exterior and inner volume 122. However, the electrostatic field 20, the ionized gas 52 and the charged biocide spray effectively combine with one another to kill the biological cells 18 whether by rupturing the cells 18, chemically attacking the cells 18, or a combination thereof. In certain embodiments, the charged biocide spray entering the inner volumes 122 of the biological cells 18 may operate to kill the biological cells 18 after the pores 126 in the cell membrane 120 have closed. The biological cells 18 are killed by the charged biocide spray, as indicated by block 190.

FIG. 11 is a schematic flow diagram illustrating the method of FIG. 10 of applying the discharge 16 having an electrostatic field 20, an ionized gas 52, and a charged biocide spray 192 to kill a biological cell 18. As shown, after the discharge 16 having the electrostatic field 20, the ionized gas 52, and the charged biocide spray 192 is created, the discharge 16 is applied to a biological cell 18. Specifically, the discharge 16 is applied to a cell membrane 120 of the biological cell 18, which surrounds an inner volume 122 of the biological cell 18. As will be appreciated, the cell membrane 120 protects the inner volume 122 of the cell from a surrounding environment 124. Thereafter, the electrostatic field 20, the ionized gas 52, and the charged biocide spray 192 of the discharge 16 cause the cell membrane 120 of the biological cell 18 to porate. More particularly, pores 126 form in the cell membrane 120 of the biological cell 18. The pores 126 cause the inner volume 122 of the biological cell 18 to become accessible by the discharge 16. More specifically, the ionized gas 52 and the charged biocide spray 192 penetrate the cell membrane 120 of the biological cell and enter the inner volume 122 of the biological cell, as shown. The pores 126 expose the inner volume 122 of the biological cell 18 to the surrounding environment 124. In certain embodiments, the application of the electrostatic field 20, the ionized gas 52, and the charged biocide spray 192 of the discharge 16 to the cell membrane 120 and the inner volume 122 of the biological cell 18 may not cause over poration of the cell membrane 120. In such circumstances, the charged biocide spray 192 entering the inner volume 122 of the biological cell 18 operates to kill the biological cells 18 after the pores 126 in the cell membrane 120 have closed, as shown. The presence of the charged biocide spray 192 within the inner volume 122 of the biological cell 18 results in the biological cell 18 dying and becoming a dead biological cell 130.

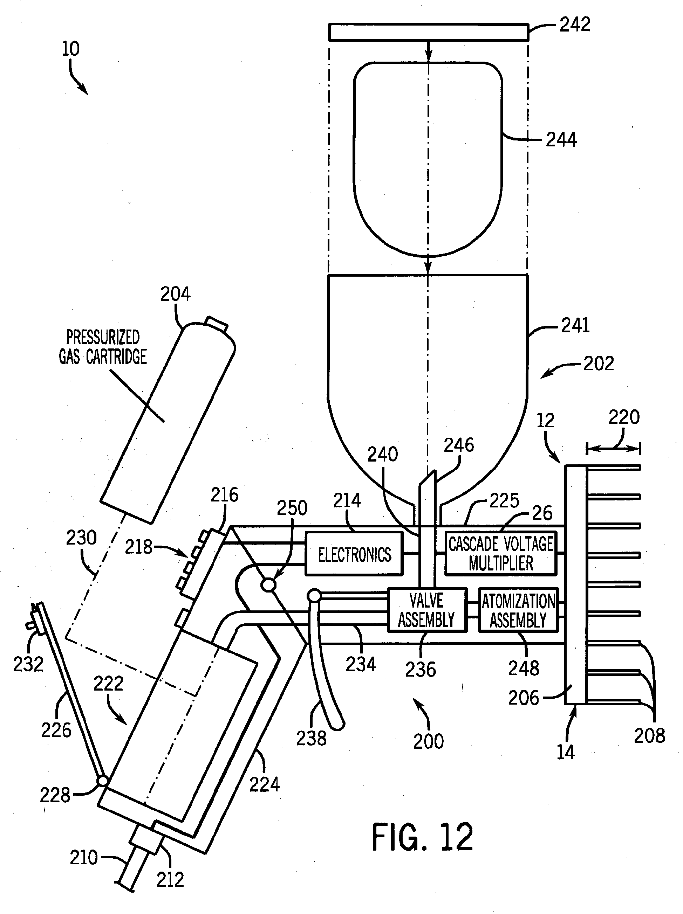

Referring now to FIG. 12, an example embodiment of an electrostatic disinfectant tool system 10 is shown. Specifically, the illustrated embodiment includes an electrostatic disinfectant tool gun 200 having an electrostatic applicator 12, a gravity applicator 202, and a pressurized gas cartridge 204. The electrostatic disinfectant tool gun 200 is configured to create a discharge 16 having an electrostatic field 20, an ionized gas 52, a charged liquid spray 72, a charged biocide spray 192, or a combination of these. As shown, the electrostatic applicator 12 includes an electrostatic field diffuser 14. The electrostatic field diffuser 14 is configured to apply the electrostatic field 20 over an area or object to be disinfected. For example, the electrostatic field diffuser 14 may be configured to apply the electrostatic field 20 at a distance of greater than approximately 5, 10, 15, 20, 25, 30, 35, or 40 centimeters from the surface or object to be disinfected.

The electrostatic field diffuser 14 comprises a plate 206 and one or more electrodes 208. The plate 206 may be of any suitable shape, such as circular, square, rectangular, triangular, polygonal, oval, or any other suitable shape. The illustrated plate 206 is flat; however, in other embodiments, the plate 206 may be curved or angled as discussed in further detail below. The plate 206 of the may be of any of a variety of sizes. The number, size, and arrangement of electrodes 208 also may vary from one implementation to another. For example, the number of electrodes 208 may be approximately 1 to 1000 or more. In the illustrated embodiment, the electrodes 208 represent an electrode grid, which may include hundreds or thousands of electrodes 208. However, it should be appreciated that the electrodes 208 may be provided in a variety of arrangements and configurations. As shown, the electrodes 208 extend a length 220 from the plate 206. For example, the length 220 may equal between approximately 0.1 to 10 centimeters, 0.5 to 5 centimeters, or any suitable length. Furthermore, the electrodes 208 may extend perpendicular to the plate 206 of the electrostatic diffuser 14, as shown, or the electrodes 208 may extend from the plate 206 at an acute angle (e.g., 10, 20, 30, 40, 50, 60, 70, or 80 degrees).

In the illustrated embodiment, power is provided to the electrostatic disinfectant tool gun 200 through an external power cable 210, which is connected to the electrostatic disinfectant tool gun 200 by an adapter 212. As will be appreciated, the external power cable 210 connects the electrostatic disinfectant tool gun 200 to an external power source, such as an electric generator or the power grid. As shown, the power cable 210 supplies power to an electronics assembly 214 in the electrostatic disinfectant tool gun 200. The electronics assembly 214 includes the monitor system 30 and/or the control system 32 described above. The electronics assembly 214 may be electrically coupled to a control panel 216. In certain embodiments, the control panel 216 may be included in the user interface 34 described above. For example, the control panel 216 may includes buttons, switches, knobs, dials, and/or a display (e.g., a touch screen) 218, which enable a user to adjust various operating parameters of the electrostatic disinfectant tool gun 200 and turn on/off the electrostatic disinfectant tool gun 200.

The electronics assembly 214 provides power to a cascade voltage multiplier 26. As described above, the cascade voltage multiplier 26 receives power from a power source (e.g., the external power cable 210 in the illustrated embodiment) and produces a high voltage power, which is supplied to the electrostatic field diffuser 14. In certain embodiments, the control panel 216 may be used to vary the power between an upper and lower limit. For example, in certain embodiments, the high power voltage may be variable between approximately 10 to 200kv, 10 to 150kV, or 10 to 100kV. However, the high power voltage may be variable or fixed to a level effective to porate and/or over-porate biological cells at a particular distance. Accordingly, the high voltage power may be at least approximately 40, 50, 60, 70, 80, 90, or 100kV. In some embodiments, the control panel 216 enables a user to adjust a distance setting, which automatically adjusts the high power voltage to an appropriate level to porate or over-porate the biological cells from the distance specified by the user. As mentioned above, the electrostatic diffuser 14 uses the high power voltage from the cascade voltage multiplier 26 to output an electrostatic field 20 over the surface or object to be disinfected. Specifically, the high power voltage may be applied to the plate 206 and the electrodes 208 of the electrostatic diffuser.

The illustrated example of FIG. 12 includes a pressurized gas cartridge 204. As will be appreciated, the pressurized gas cartridge 204 serves as the gas supply 54 and/or gas supply 80 described above. Specifically, the pressurized gas cartridge 204 provides a gas flow for the production of the ionized gas 52, the charged liquid spray 72 and/or the charged biocide spray 192. For example, the pressurized gas cartridge 204 may include nitrogen, carbon dioxide, or air. In the illustrated example, the pressurized gas cartridge 204 is disposed inside a gas mount 222 (e.g., receptacle) of a handle 224 of the electrostatic disinfectant tool gun 200. In another embodiment, the pressurized gas cartridge 204 may be disposed in a barrel 225 of the electrostatic disinfectant tool gun 200. In either embodiment, the gas mount 222 may be accessed by opening a door 226. The illustrated door 226 is coupled to the handle 224 of the electrostatic disinfectant tool gun 200 by a hinge 228, allowing the door to rotate open. With the door 226 open, as shown, the pressurized gas cartridge 204 may be placed inside the gas mount 222 of the handle 224, as indicated by the line 230. After the pressurized gas cartridge 204 is placed inside the gas mount 222 of the handle 224, the door 226 may be closed and releaseably secured to the handle 224 by a latch 232.

With the pressurized gas cartridge 204 placed within the electrostatic disinfectant tool gun 200, the pressurized gas cartridge 204 provides gas to the electrostatic applicator 12. As shown, the electrostatic disinfectant tool gun 200 includes a gas passage 234, which connects the pressurized gas cartridge 204 in the handle 224 to a valve assembly 236. The valve assembly 236 may be further linked to a trigger assembly 238. As will be appreciated, a user may actuate the trigger assembly 238, which initiates a gas flow from the pressurized gas cartridge 204 through the valve assembly 236. Furthermore, a liquid passage 240 is coupled to the valve assembly 236. The liquid passage 240 may be further coupled to the gravity applicator 202.

The gravity applicator 202 serves as the liquid supply 82 discussed above. More specifically, a liquid may be disposed within the gravity applicator 202 for use in generating a liquid spray. For example, the liquid disposed within the gravity applicator 202 may be water for use in generating a charged water spray 72, or a biocide for generating a charged biocide spray 192. In the illustrated embodiment, the gravity applicator 202 has a cup portion 241 and a lid 242. The cup portion 24 is configured to receive a resilient container, such as a liquid pouch 244. The liquid pouch 244 may be disposed inside the cup portion 241 of the gravity applicator 202 and contact the liquid passage 240. In particular, the liquid pouch 244 may contact a sharp edge 246 of the liquid passage 240. In operation, the contact between the sharp edge 246 of the liquid passage 240 and the liquid pouch 244 may cause the sharp edge 246 to pierce the liquid pouch 244. As will be appreciated, the piercing of the liquid pouch 244 by the sharp edge 246 will allow the liquid within the pouch to pass through the liquid passage 240 to the valve assembly 236. In other embodiments, instead of inserting the liquid pouch 244 into the cup portion of the gravity applicator 202, a liquid may be poured into the cup portion 241 of the gravity applicator 202, and the lid 242 may be placed on top of the cup portion 241 to contain the liquid. In certain embodiments, the resilient container (e.g., pouch 244) may be a sealed bag made of plastic, rubber, foil, paper, or another material. In other embodiments, the gravity applicator 202 receives a rigid container, such as a box, can, or cup, which may be made of metal, plastic, or paper.

During operation, a user may actuate the trigger assembly 238, which initiates a gas flow from the pressurized gas cartridge 204 through the valve assembly 236. In addition, the actuation of the trigger assembly 238 initiates a fluid flow from the liquid pouch 244 in the gravity applicator 202 through the valve assembly 236. The gas and fluid flow pass towards an atomization assembly 248. The atomization assembly 248 uses the gas from the pressurized gas cartridge 204 to atomize the liquid supplied by the gravity applicator 202 to generate a spray. As discussed in detail below, the spray generated by the atomization assembly 248 passes through the electrostatic applicator 12 to generate a charged liquid spray 72, such as a charged biocide spray 192.

The illustrated embodiment of the electrostatic disinfectant tool gun 200 further includes a pivot assembly 250 between the handle 224 and the barrel 225. As will be appreciated, the pivot assembly 250 enables rotation of the handle 224 relative to the barrel 225, such that the user can selectively adjust the configuration of the electrostatic disinfectant tool gun 200 between a straight configuration and an angled configuration. As illustrated, the electrostatic disinfectant tool gun 200 is arranged in the angled configuration, wherein the handle 224 is angled crosswise to the barrel 225. The ability to manipulate the electrostatic disinfectant tool gun 200 in this manner assists the user in applying the discharge in various applications. That is, different configurations of the electrostatic disinfectant tool gun 200 may be more convenient or appropriate for applying the discharge in different environments or circumstances.

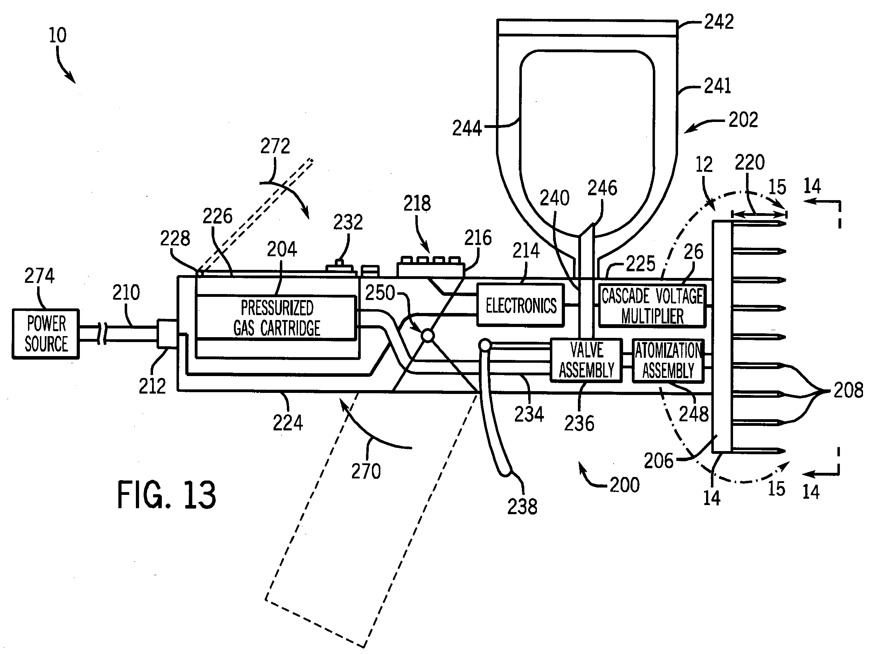

Referring now to FIG. 13, the electrostatic disinfectant tool system 10 of FIG. 12 is shown in the straight configuration with the handle 224 substantially in-line with the barrel 225. In particular, the handle 224 and the barrel 225 are substantially parallel with one another, and disposed end to end, such that the electrostatic disinfectant tool gun 200 has the straight configuration. The straight configuration of FIG. 13 may be beneficial in tight spaces, where the angled configuration, as shown in FIG. 12, may not fit as well. The illustrated embodiment shows the handle 224 of the electrostatic disinfectant tool gun 200 rotated about the pivot assembly 250 in a direction 270. Additionally, the pressurized gas cartridge 204 is disposed inside the handle 224 of the electrostatic disinfectant tool gun 200 with the door 226 of the handle 224 closed, as indicated by reference numeral 272, and secured with the latch 232. Furthermore, the liquid pouch 244 is disposed inside the gravity applicator 202 with the lid 242 disposed on top of the gravity applicator 202. As shown, the sharp edge 242 of the liquid passage 240 punctures the liquid pouch 244, allowing the liquid within the liquid pouch 244 to flow through the liquid passage 240 to the valve assembly 236. Further, the electrostatic disinfectant tool system 10 includes a power source 274 connected to the external power cable 210. In various alternative embodiments, the power source 274 may be a battery, an electrical generator, or a power grid.

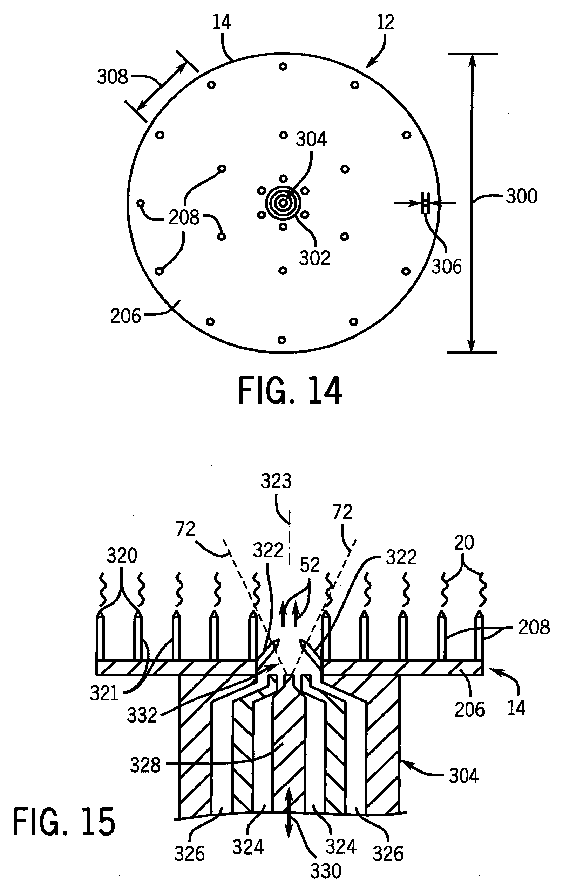

FIG. 14 is a partial front view, taken along line 14-14 of FIG. 13, of the electrostatic disinfectant tool gun 200 illustrated in FIG. 13. As discussed above, the electrostatic diffuser 14 of the applicator 12 is configured to receive a high voltage power from the cascade voltage multiplier 26 and distribute an electrostatic field 20 over a surface or object to be disinfected. In the illustrated example, the electrostatic diffuser 14 includes the plate 206 and electrodes 208. As shown, the plate 206 of the electrostatic diffuser 14 has a circular configuration and has a diameter 300, which may be between approximately 1 to 100, 5 to 75, 10 to 50, 20 to 40, or 25 to 35 centimeters. However, the diameter 300 of the plate 206 may be selected based on a target object, a target distance, a voltage level, and other parameters of the electrostatic disinfectant tool gun 200. Furthermore, although the plate 206 is illustrated as a circular plate 206, the plate 206 may be square, rectangular, triangular, polygonal, oval, or any other suitable shape. The plate 206 further includes an aperture 302 located generally at the center of the plate 206. As discussed in detail below, the aperture 302 allows the ionized gas 52, charged liquid spray 72 and/or charged biocide spray 192 to pass from a nozzle 304 of the electrostatic applicator 12 to the area to be disinfected.

The electrostatic diffuser also includes the electrodes 208. In some embodiments, the electrodes 208 have a generally cylindrical shape and may be constructed from a nickel and chromium alloy or a nickel and titanium alloy. In the illustrated example, the electrodes 208 each have a diameter 306. For example, the diameter 306 of each electrode 208 may be approximately 0.1 to 5, 0.5 to 3, or 1 to 2 millimeters. In certain embodiments, the diameter 306 may be less than approximately 0.1, 0.5, 1, 1.5, or 2 millimeters. As will be appreciated, the electrodes 208 may be flexible or resilient due at least in part to the relatively small diameter 306, and the substantially greater length 220 compared with the diameter 306. As discussed below, in certain embodiments, the electrodes 208 may have a sharp edge or point at the tip of each electrode 208. Furthermore, the electrodes 208 are generally spaced at an offset distance 308 from each other. The distance 308 may be between approximately 0.1 to 5, 0.5 to 3, or 1 to 2 millimeters. In certain embodiments, the distance 308 may be less than approximately 0.1, 0.5, 1, 1.5, 2, 2.5, or 3 millimeters. However, the shape, material construction, diameter 306, length 220, and offset distance 308 may vary from one implementation to another. Accordingly, certain embodiments of the electrodes 208 may be made of various conductive materials, various shapes (e.g., rectangular, oval, or flat), and various dimensions effective to produce the electrostatic field 20.

FIG. 15 is a partial cross-sectional side view of the electrostatic disinfectant tool gun 200 of FIG. 13, taken within line 15-15 of FIG. 13, illustrating an embodiment of the electrostatic applicator 12. As shown in FIG. 15, the applicator 12 includes the electrostatic diffuser 14 and the nozzle 304. In certain embodiments, the nozzle 304 may be included in the atomization assembly 248. As shown, the electrostatic diffuser 14 includes the plate 206 and the electrodes 208, which are configured to emit the electrostatic field 20. Each electrode 208 is an elongated structure, such as thin protruding shaft, that extends outwardly from the plate 206 to a sharp edge 320. As will be appreciated, the sharp edge 320 may improve the generation and application of the electrostatic field 20 to the surface or object to be disinfected. As illustrated in FIG. 15, the electrodes 208 include electrodes 321 and electrodes 322, which may be angled differently from one another. For example, the illustrated electrodes 321 may be angled approximately 90 degrees to the plate 206, while the electrodes 322 may be angled less than 90 degrees to the plate 206. As illustrated in FIG. 15, the electrodes 322 are angled inwardly toward an axis 323 of the electrostatic disinfectant tool gun 200, such that the electrodes 322 extend over the aperture 302 to ionize the gas supplied by the pressurized gas cartridge 204 and/or charge the liquid supplied by liquid pouch 244 in the gravity adaptor 202. For example, the electrodes 322 may be angled less than approximately 10, 20, 30, 40, 50, 60, 70, or 90 degrees relative to the axis 323, such that the electrodes 322 extend directly into a liquid spray region.

As shown, the nozzle 304 includes a gas passage 324 and a liquid passage 326. The nozzle 304 also includes a needle valve 328. As will be appreciated, the needle valve 328 may be included in the valve assembly 236. The gas passage 324 is configured to receive a gas flow from a gas supply, such as the pressurized gas cartridge 204. Additionally, the liquid passage 326 is configured to receive a liquid flow from a liquid supply, such as the liquid pouch 244 in the gravity applicator 202. The needle valve 328 may be actuated, as indicated by the arrow 330, allowing the liquid flow in the liquid passage 326 and the gas flow in the gas passage 324 to combine to form a spray at a mouth 332 of the nozzle 304. Additionally, the nozzle 304 may be configured to flow gas at the mouth 332 of the nozzle 304. In certain embodiments, the needle valve 328 may be actuated by the trigger assembly 238 of the electrostatic disinfectant tool gun 200. The gas and spray may exit the nozzle 304 and pass through the aperture 302 of the electrostatic diffuser 14. In the illustrated embodiment, the gas and spray may pass over the electrodes 322 allowing the gas and spray to absorb an electric charge from the electrostatic field 20, thereby generating the ionized gas 52 and the charged liquid spray 72, respectively.

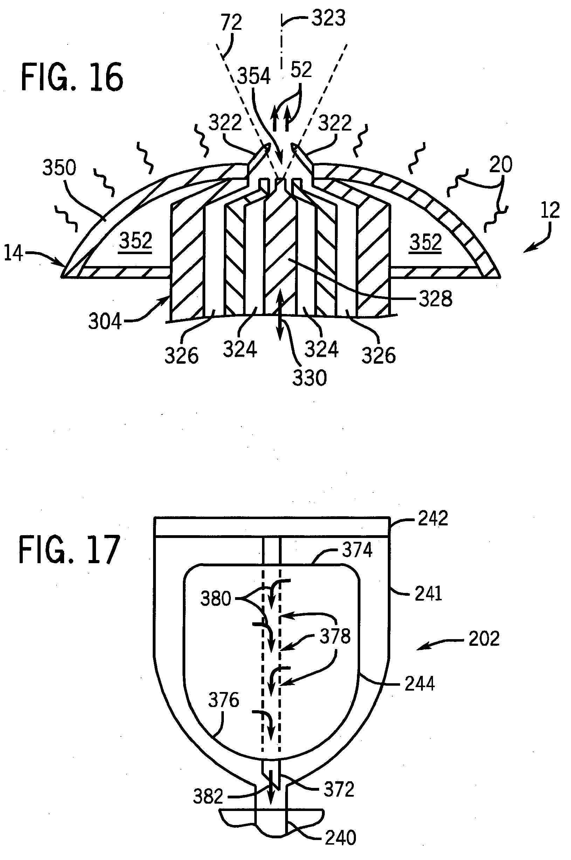

FIG. 16 is a partial cross-sectional side view of the electrostatic disinfectant tool gun 200 of FIG. 13, taken within line 15-15 of FIG. 13, illustrating another embodiment of the electrostatic applicator 12. As shown, the electrostatic applicator 12 includes elements and element numbers similar to the electrostatic applicator 12 provided in FIG. 15. As illustrated in FIG. 16, the electrostatic diffuser 14 of this example has a dome-shaped configuration. The electrostatic diffuser 14 has an outer wall 350 and a hollow interior 352. The electrostatic diffuser 14 also has an aperture 354 configured to allow the spray and/or the gas to flow from the nozzle 304 to the surface or object to be disinfected. As shown, the electrostatic diffuser 14 includes the electrodes 322 extending at an angle from the outer wall 350 and about the aperture 354. The gas and/or the spray supplied by the electrostatic disinfectant tool gun 200 may pass from the nozzle 304, through the aperture 354, and across the electrodes 322, thereby absorbing an electric charge from the electrostatic field 20 to create the ionized gas 52 and/or the charged liquid spray 72.

FIG. 17 is a cross-sectional side view of an embodiment of the gravity applicator 202. As shown, the gravity applicator 202 includes the cup portion 241 and the lid 242. Additionally, the gravity applicator 202 is configured to receive the liquid pouch 244, which may be disposed in the cup portion 241 of the gravity applicator 202. As illustrated in FIG. 17, the lid 242 of the gravity applicator 202 includes a tube portion 370 that, when the lid 242 is placed on the cup portion 241, extends downward into the gravity applicator 202. Furthermore, the tube portion 370 includes a sharp edge 372, which may pierce the liquid pouch 244 as the lid 242 is placed onto the cup portion 241 of the gravity applicator 202. Specifically, the tube portion 370 may be sufficiently long that is may pierce a top surface 374 of the liquid pouch 244, extend through the liquid pouch 244, and subsequently pierce a bottom surface 376 of the liquid pouch 244. The tube portion 370 may also include perforations 378. As a result, the liquid within the liquid pouch 244 may pass through the perforations 378, as indicated by arrows 380, and down the tube portion 370. The liquid may then pass through an opening 382 of the tube portion 370 and flow to the liquid passage 240 of the electrostatic disinfectant tool gun 200.

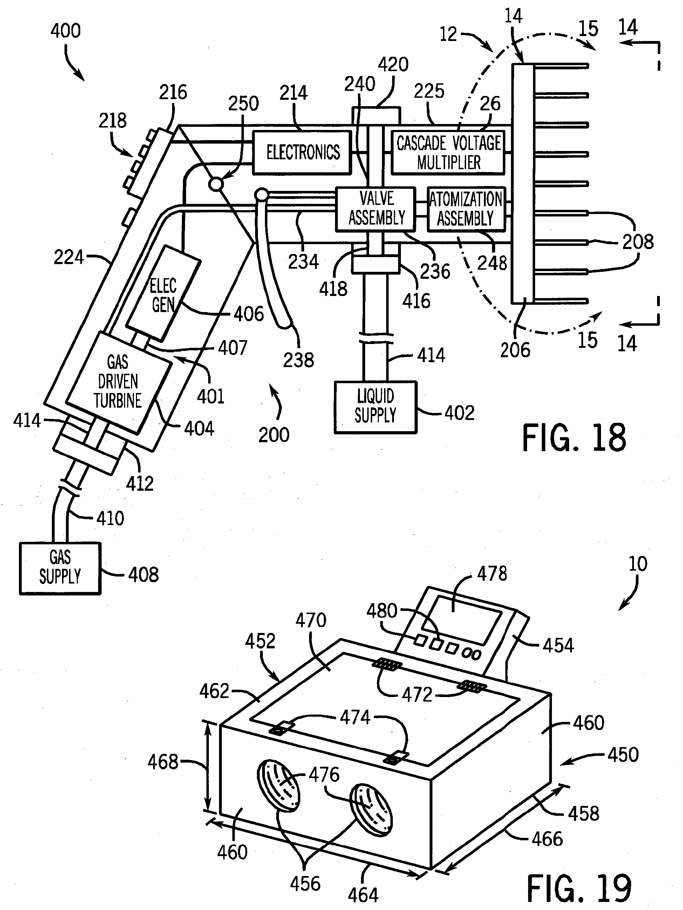

Referring now to FIG. 18, another embodiment of an electrostatic disinfectant tool system 400 is shown. The electrostatic disinfectant tool system 400 of FIG. 18 includes elements and element numbers similar to the electrostatic disinfectant tool system 10 provided in FIG. 12. However, instead of a pressurized gas cartridge 204, the electrostatic disinfectant tool system 400 of FIG. 18 includes a gas-driven turbine system 401. Also, instead of having a gravity applicator 202, the electrostatic disinfectant tool system 400 of FIG. 18 includes a liquid supply 402.

The gas-driven turbine system 401 includes a gas driven turbine 404 and an electrical generator 406. As shown, the gas driven turbine 404 and the electrical generator 406 are disposed inside the handle 224 of the electrostatic disinfectant tool gun 200. The gas driven turbine 404 is configured to receive a gas flow from a gas supply 408. For example, the gas supply 408 may be a pressurized gas tank, and may include nitrogen, oxygen, carbon dioxide air, or another gas. The gas may flow from the gas supply 408 through a connector 410, which is connected to the handle 224 of the electrostatic disinfectant tool gun 200 by an adapter 412. The gas flows from the gas supply 408 through the connector 410 and through a gas passage 411 to the gas driven turbine 404. In the gas driven turbine 404, the gas flow drives a plurality of turbine blades to rotate a shaft 407. The gas flow continues to the valve assembly 236 through the gas passage 234. The electrical generator 406 may be mechanically coupled to the gas driven turbine 404 via the shaft 407. As a result, the gas-driven turbine 404 transfers rotational energy to the electrical generator 406, which converts the rotational energy into electrical energy. As will be appreciated, the power generated by the electrical generator 406 may be used to power the electrostatic disinfectant tool gun 200. Specifically, the electrical generator 406 may be electrically coupled to the electronics assembly 214, which provides power to the cascade voltage multiplier 26 and the control panel 216, as described above.

As illustrated in FIG. 18, the electrostatic disinfectant tool system 400 includes the liquid supply 402. The liquid supply 402 is connected to the electrostatic disinfectant tool gun 200 by a connector 414 and an adaptor 416. The liquid supply 402 may include a liquid pump coupled to a supply tank, a pressurized liquid tank, pressurized liquid bottle, or another type of liquid supply system. Furthermore, the liquid supply 402 may be stationary or portable. The liquid supply 402 provides a liquid flow, such as water or biocide, to the electrostatic disinfectant tool gun 200 for use in creating a liquid spray. As shown, the liquid supply 402 provides a liquid flow through the connector 414 and a liquid passage 418 to the valve assembly 236 of the electrostatic disinfectant tool gun 200. The electrostatic disinfectant tool system 400 of FIG. 18 also includes a cap 420, which may be releaseably secured to the electrostatic disinfectant tool gun 200. Specifically, the cap 420 may be secured to the electrostatic disinfectant tool gun 200 in place of the gravity applicator 202, covering and sealing the liquid passage 240. As will be appreciated, the cap 420 may be removed for applications in which an operator uses the gravity applicator 202 to provide a liquid flow to the electrostatic disinfectant tool gun 200.

Referring now to FIGS. 19, 20, and 21, an example stationary electrostatic disinfectant tool unit 450 is shown. As best illustrated in FIG. 19, the stationary electrostatic disinfectant tool unit 450 includes a chamber 452, a control panel 454 and hand inserts 456. The electrostatic disinfectant tool unit 450 is configured to receive a user's hands, tools, utensils, instruments, or other objects to be disinfected. As discussed in detail below, the electrostatic disinfectant tool unit 450 disposes a discharge 16 within the chamber 452 to disinfect the objects placed inside the electrostatic disinfectant tool unit 450. As shown, the electrostatic disinfectant tool unit 450 has a generally box-shaped configuration with a base 458, sides 460, and a top 462. Further, the electrostatic disinfectant tool unit 450 has a width 464, a depth 466, and a height 468. For example, the width 464, the depth 466, and the height 468 may be approximately 10 to 100, 20 to 80, or 30 to 60 centimeters. However, the particular dimensions may vary depending on the target application, expected size of devices being disinfected in the chamber 452, and so forth.

The top 462 of the electrostatic disinfectant tool unit 450 has a lid 470, which is secured to the top 462 of the electrostatic disinfectant tool unit 450 by hinges 472 and latches 474. As will be appreciated, the latches 474 may be released and the lid 470 may be opened, rotating about the hinges 472. When the lid 470 is opened by a user, objects to be disinfected may be placed within the chamber 452 of the electrostatic disinfectant tool unit 450. Once the objects to be disinfected are placed inside the chamber 452, the lid 470 may be closed and the latches 474 may be secured to the top 462 of the electrostatic disinfectant tool unit 450. In certain embodiments, the lid 470 may be constructed of a clear or transparent material, such as plastic or glass, to allow a user to view the objects as they are being disinfected in the electrostatic disinfectant tool unit 450.

As mentioned above, the electrostatic disinfectant tool unit 450 includes hand inserts 456. The hand inserts 456 may be generally circular or oval openings in the side 460 of the electrostatic disinfectant tool unit 450. The hand inserts 456 may further include linings 476 configured to receive a user's hands. For example, the linings 476 may be constructed from rubber or plastic. As objects are disinfected within the electrostatic disinfectant tool unit 450, as user may place their hands through the inserts 456 and manipulate the tools inside the electrostatic disinfectant tool unit 450 during the disinfection process. As will be appreciated, the linings 476 serve to protect the user's hands from exposure to the electrostatic field 20, ionized gas 52, charged liquid spray 72 and/or charged biocide spray 192. For example, the linings 476 may be resilient sleeves, which extend into the chamber 452 and completely seal the chamber 452 from the exterior environment. In certain embodiments the linings 476 may be a resilient layer of a polymer or an elastomer, such as rubber. The linings 476 also may be electrically insulative and chemical resistant. In some embodiments, the linings 476 may include an electrical insulation layer, a chemical resistant layer, a moisture resistant layer, or any combination thereof.

As mentioned above, the electrostatic disinfectant tool unit 450 includes a control panel 454. As shown, the control panel 454 includes a display 478 and buttons, knobs, or dials 480. The control panel 454 is configured to enable a user to adjust various settings and operating parameters of the electrostatic disinfectant tool unit 450. For example, the display 478 may communicate system feedback, such as the flow rate of the charged liquid spray 78 or ionized gas 52, the voltage of the electrostatic field 20, or other system information. Additionally, the buttons, knobs, or dials 480 may be configured to allow the user to control or adjust the operation of the electrostatic disinfectant tool unit 450. For example, the buttons, knobs, or dials 480 may be used to adjust the voltage of the electrostatic field 20 or the flow rate of the charged fluid spray 72 or the ionized gas 52. In certain embodiments, the display 478 is a flat panel display, such as a liquid crystal display (LCD) and/or a touch screen. Thus, the touch screen display 478 may enable both user input (e.g., interactive menus) and display of various system information.

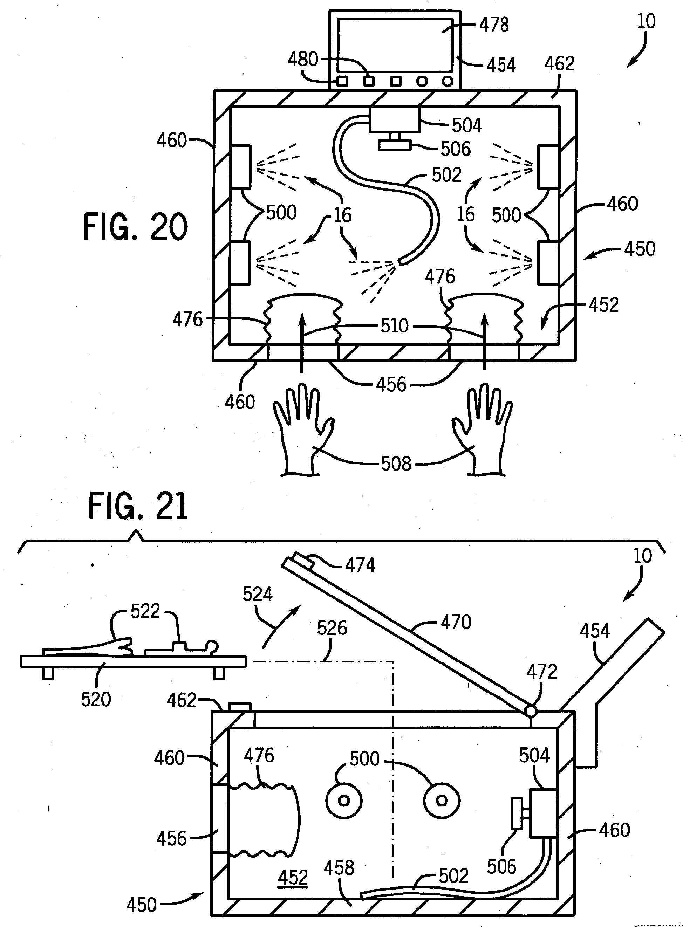

FIG. 20 is a top view of the example electrostatic disinfectant tool unit 450 of in FIG. 19. In FIG. 20, the electrostatic disinfectant tool unit 450 is shown with the top 462 removed, exposing the interior of the chamber 452. As shown, the chamber 452 includes discharge jets 500 and a discharge applicator 502. The discharge jets 500 are configured to emit the discharge 16, which may include the electrostatic field 20, the ionized gas 52, and/or the charged liquid spray 72. Moreover, the discharge applicator 502 allows a user to manually direct a flow of the discharge 16 during operation of the electrostatic disinfectant tool unit 450. Specifically, the user may use the discharge applicator 502 to apply the discharge 16 in a specific location or area of an object within the chamber 452. The discharge applicator 502 is connected to a discharge output 504 having a discharge output valve 506. The discharge output valve 506 may be operated by a user to control the flow rate of the discharge 16 during operation of the electrostatic disinfectant tool unit 450. FIG. 20 illustrates the insertion of a user's hands 508 into the electrostatic disinfectant tool unit 450 via the linings 476. Specifically, the user's hands 508 may be inserted through the hand inserts 456 into the linings 576 in a direction 510. As illustrated, the linings 476 create a sealed barrier between the exterior environment and the chamber 452, and may be extended to any suitable distance into the chamber 452. The linings 476 are shown substantially compressed toward the hand inserts 456, yet the linings 476 may be expanded (e.g., unfolded and/or stretched) further into the chamber 452.

FIG. 21 is a cross-sectional side view of the example electrostatic disinfectant tool unit 450 of FIG. 19. As illustrated in FIG. 21, the electrostatic disinfectant tool unit 450 includes a tray 520, which may be placed inside the chamber 452 of the electrostatic disinfectant tool unit 450. Specifically, the tray 520 may support a plurality of objects, such as tools and instruments 522, to be disinfected in the chamber 452. For example, the instruments 522 may include medical instruments (e.g., surgical instruments), food instruments (e.g., cooking utensils), or other types of objects. After opening the lid 470 of the electrostatic disinfectant tool unit 450, as indicated by arrow 524, the tray 520 may be inserted into the chamber 452 of the electrostatic disinfectant tool unit 450, as indicated by line 526. Thereafter, the lid 470 may be closed, and the electrostatic disinfectant tool unit 450 may be operated by the user to disinfect the tools and instruments 522.

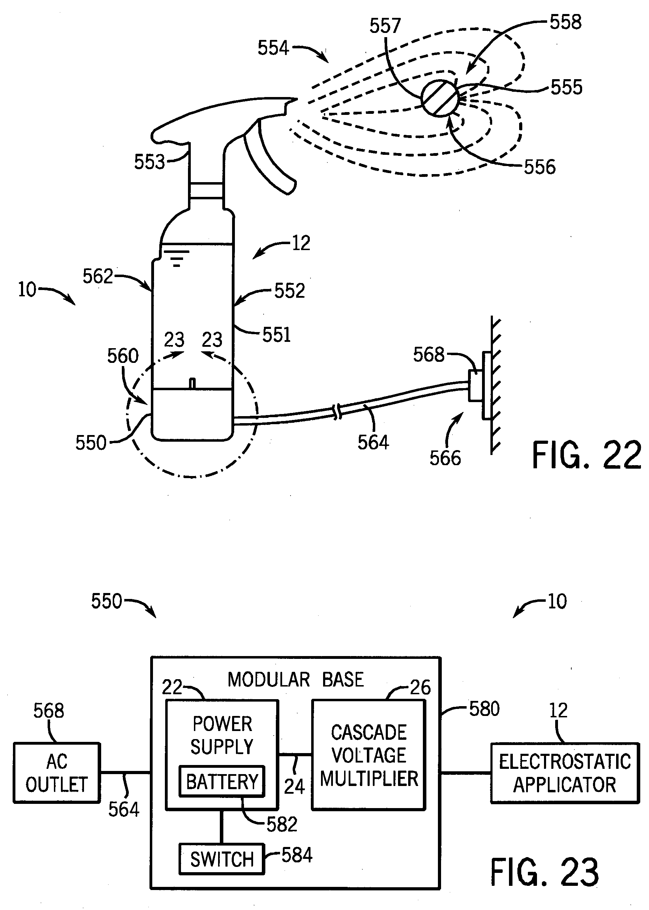

FIG. 22 is a schematic of an example embodiment of the electrostatic disinfectant tool system 10. In the illustrated embodiment, the electrostatic disinfectant tool system 10 includes an electrostatic module 550. More specifically, the electrostatic module 550 is coupled to the electrostatic applicator 12, which may be a spray bottle 552, as shown, or other applicator configured to emit a spray of liquid. For example, the electrostatic applicator 12 may be an off-the-shelf spray bottle 552 or a customized spray bottle 552 configured to engage with the electrostatic applicator 12. Similarly, the electrostatic applicator 12 may be a reusable spray bottle 552 or a disposable spray bottle 552, such as a plastic spray bottle made of a transparent or translucent plastic. As appreciated, the spray bottle 552 may include a bottle portion 551 and a spray head portion 553 coupled to the bottle portion 551. As discussed in detail below, the electrostatic module 550 is configured to provide an electrostatic charge to the liquid contained in the spray bottle 552. For example, the spray bottle 552 may contain water, disinfectant, biocide, insecticide, herbicide, or other liquid. The electrostatically-charged liquid may then be applied to a surface or instrument to be disinfected. As discussed above, the electrostatic charge of the liquid assists the application of the liquid to the surface or instrument to be disinfected. For example, in the illustrated embodiment, a spray 554 is emitted from the spray bottle 552 towards an object 556 to be disinfected. Due to the electrostatic charge of the spray 554, the spray 554 is more effectively applied to the object 556. For example, a portion 558 of the object 556 opposite the spray bottle 552 may experience increased coverage of the spray 554 as a result of the electrostatic charge applied to the spray 554. In particular, the electrostatically charged spray 554 wraps around the object 556, thereby providing improved coverage on a rear 555 of the object 556 opposite a front 557 of the object 556 facing the spray head portion 553. In other words, the electrostatically charged spray 554 provides 360 degree coverage around the object 556 by virtue of the wrapping effect achieved by the electrostatic module 550.

In the illustrated embodiment, the electrostatic module 550 is coupled to a base 560 of the spray bottle 552. In other embodiments, the electrostatic module 550 may be coupled to a side 562 of the spray bottle 552. As mentioned, the electrostatic module 550 is configured to provide an electrostatic charge to the liquid contained in the spray bottle 552. The electrostatic module 550 may be powered by a grounded battery or an external power outlet. Additionally, the electrostatic module 550 includes a cable 564, which is connected to an electrical ground 566. Specifically, in the illustrated embodiment, the cable 564 is coupled to an AC outlet 568. In certain embodiments, the cable 564 may also transfer power from the AC outlet 568 to the electrostatic module 550.

FIG. 23 is a schematic of an example embodiment of the electrostatic module 550. As discussed above, the electrostatic module 550 is coupled to the electrostatic applicator 12, which may be the spray bottle 552. Specifically, the electrostatic module 550 includes a module base 580 which houses the various components of the electrostatic module 550. The module base 580 may be coupled to the electrostatic applicator 12 with a threaded connection, compression connection, snapping joint, or other connection. Additionally, other fasteners may be used to secure the modular base 580 to the electrostatic applicator 12, such as adhesives, screws, Velcro, rubber straps, latches, and so forth.

As shown, the module base 580 houses the power supply 22 and the cascade voltage multiplier 26 of the electrostatic disinfectant tool system 10. As discussed above, the cascade voltage multiplier 26 receives the power 24 from the power supply 22 and converts the power 24 to higher voltage power. The higher voltage power is then applied to the liquid within the electrostatic applicator 12, e.g., the spray bottle 552, using an electrode, thereby electrostatically charging the liquid. For example, the cascade voltage multiplier 26 may apply approximately 5 to 20, 8 to 16, or 10 to 14 kV to the liquid within the spray bottle 552. As mentioned above, the power supply 22 of the electrostatic module 550 include a grounded battery 582. For example, the battery 582 may be a disposable battery or a rechargeable battery (e.g., a 9V battery) housed within the modular base 580. In such an embodiment, the battery is coupled to an electrical ground, such as the AC outlet 568, by the cable 564. Alternatively, the power supply 22 may be coupled to an external power source, such as a 120 volt power outlet, by the cable 564. The electrostatic module 550 also includes a switch 584 coupled to the power supply 22. As will be appreciated, the switch 584 may be activated by a user to provide power from the power supply 22 to the cascade voltage multiplier 26, thereby electrostatically charging the liquid within the electrostatic applicator, e.g., the spray bottle 552. Similarly, the switch 584 may be disengaged to stop the power supply 22 from providing power to the cascade voltage multiplier 26. In certain embodiments, the switch 584 may be a rocker switch, toggle switch, button, or other switch.

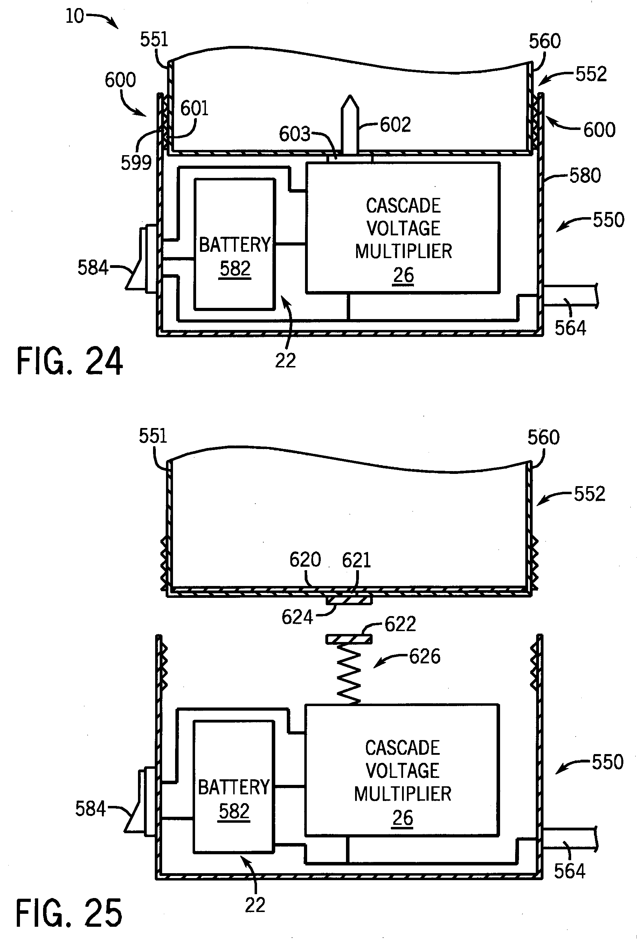

FIG. 24 is a schematic of an example embodiment of the electrostatic module 550, illustrating the electrostatic module coupled to the base 560 of the spray bottle 552. As illustrated, the electrostatic module 550 is secured to the base 560 of the spray bottle 552 by a threaded connection 600 (e.g., mating threads 599 and 601). In particular, as the electrostatic module 550 receives the base 560 of the spray bottle 552, threads 601 of the base 560 and threads 599 of the electrostatic module 550 engage with one another, thereby securing the electrostatic module 550 to the spray bottle 552.

Additionally, as the electrostatic module 550 receives the base 560 of the spray bottle 552, a push pin electrode 602 of the electrostatic module 550 pierces the base 560 of the spray bottle 552 and makes contact with the liquid inside the spray bottle 552. As shown, the push pin electrode 602 is coupled to the cascade voltage multiplier 26 of the electrostatic module 550. Therefore, the cascade voltage multiplier 26 transfers an electrostatic charge to the liquid in the spray bottle 552 through the push pin electrode 602. For example, the push pin electrode 602 may be made from copper or another electrically conductive material. Furthermore, the module 550 may include an electrode seal 603, such as a rubber O-ring seal, to seal the bottle 552 around the puncture created by the electrode 602. In certain embodiments, the seal 603 may include 1, 2, 3, 4, 5, or more concentric O-rings to ensure a watertight seal around the electrode 602.

FIG. 25 is a schematic of an example embodiment of the electrostatic module 550. In the illustrated embodiment, the base 560 of the spray bottle 552 includes a conductive plate 620 (e.g., plate-style electrode) configured to diffuse the electrostatic charge received from the electrostatic module 550 into the liquid contained in the bottle 552. Specifically, as the electrostatic module 550 is coupled to the base 560 of the spray bottle 552, an electrode plate 622 of the electrostatic module 550 makes contact with an electrode contact 624 of the spray bottle 552. As shown, the electrode contact 624 is attached to the conductive plate 620 disposed inside the base 560 of the spray bottle 552 by a conductive element 621 extending through the bottle 552. For example, the conductive plate 620, the electrode plate 622, and the electrode contact 624 may be made from a conductive metal, such as copper. Furthermore, the electrode plate 622 of the electrostatic module 550 is coupled to the cascade voltage multiplier 26 with a biasing element 626. Specifically, the biasing element 626, which may be a spring, biases the electrode plate 622 towards the electrode contact 624. In this manner, a good, solid contact can be maintained between the electrode plate 622 and the electrode contact 624, thereby providing an effective transfer of electrostatic charge from the cascade voltage multiplier 26 to the conductive plate 620. When the electrostatic charge is transferred to the conductive plate 620, the liquid within the spray bottle 552 becomes electrostatically-charged through contact with the conductive plate 620.

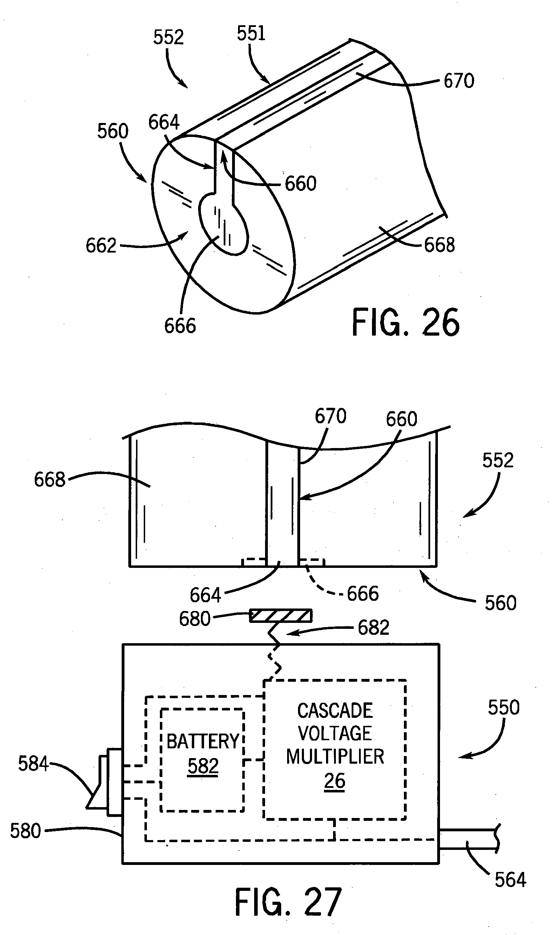

FIG. 26 is a partial perspective view of an example embodiment of the spray bottle 552, which may be configured for use with the electrostatic module 550. More specifically, in the illustrated embodiment, the bottle portion 551 of the spray bottle 552 includes a conductive material 660. That is, the bottle portion 551 includes a non-conductive portion 662 and a conductive portion 664. For example, the non-conductive portion 622 may be a non-metallic material, such as plastic, and the conductive portion 664 is formed from the conductive material 660, which may be a conductive plastic (e.g., an organic polymer that conducts electricity, a plastic impregnated with metal particles, etc.), a metal, or other conductive material 660. As shown, the non-conductive portion 662 and the conductive portion 664 are integrally formed with one another to make the bottle portion 551 of the spray bottle 552. For example, the conductive portion 664 (e.g., the conductive material 660) may be molded into the non-conductive portion 662 (e.g., a standard plastic spray bottle) to form the bottle portion 551 of the spray bottle 552. In other words, the conductive portion 664 may be a conductive piece (e.g., a metal piece) that is molded in place with the bottle portion 551 (e.g., a plastic bottle) of the spray bottle 552. As will be appreciated, the conductive material 660 is configured to transmit or transfer an electrostatic charge from the cascade voltage multiplier 26 of the electrostatic module 550, in the manner described below.

In the illustrated embodiment, a conductive base portion 666 of the base 560 of the spray bottle 552 is formed from the conductive material 660. In other embodiments, the entire base 560 of the spray bottle 552 may be formed with the conductive material 660. As discussed below, an electrode contact or plate of the electrostatic module 550 may be configured to contact the conductive base portion 666 of the base 560 that is formed from the conductive material 660 when the spray bottle 552 and the electrostatic module 550 are coupled to one another. Additionally, a sidewall 668 of the spray bottle 552 includes a conductive side portion 670 formed from the conductive material 660. As shown, the portions 666 and 670 of the spray bottle 552 formed from the conductive material 660 are coupled to one another. In this manner, the entire conductive portion 664 of the spray bottle 552 may transfer and diffuse the electrostatic charge from the cascade voltage multiplier 26 of the electrostatic module 550 to the liquid contained within the spray bottle 552.

FIG. 27 is a schematic of an example embodiment of the electrostatic module 550. Specifically, the illustrated embodiment of the electrostatic module 550 is configured to be coupled to the spray bottle 552 illustrated in FIG. 26. As the electrostatic module 550 is coupled to the base 560 of the spray bottle 552, an electrode plate 680 of the electrostatic module 550 makes contact with the conductive portion 664 of the spray bottle 552. For example, the electrode plate 680 may contact the conductive base portion 666 of the conductive portion 664 shown in FIG. 26. The electrode plate 680 is coupled to the cascade voltage multiplier 26 by a biasing element 682, which may be a spring, that biases the electrode plate 680 towards the spray bottle 552 (e.g., the conductive portion 664 of the spray bottle 552). In this manner, a strong contact can be maintained between the electrode plate 680 and the spray bottle 552 (e.g., the conductive
I portion 664 of the spray bottle 552) thereby providing an effective transfer of electrostatic charge from the cascade voltage multiplier 26 to the conductive portion 664 of the spray bottle 552. When the electrostatic charge is transferred to the conductive portion 664, the liquid within the spray bottle 552 becomes electrostatically-charged through contact with the conductive portion 664 of the spray bottle 552.

While only certain features of the invention have been illustrated and described herein, many modifications and changes will occur to those skilled in the art. It is, therefore, to be understood that the appended claims are intended to cover all such modifications and changes as fall within the scope of the invention.

## Claims

1. A system (10; 50; 70; 400), comprising:
an electrostatic tool (200; 400) configured to output a discharge to kill a biological cell by electroporation, wherein the electrostatic tool (200; 400) comprises:
an electrostatic applicator (12; 56) configured to output an electrostatic field (20), wherein the discharge (16) comprises the electrostatic field (20),
**characterized in that** the electrostatic tool (200; 400) comprises a gas passage (234) configured to flow a gas, the electrostatic applicator (12; 56) is configured to ionize the gas to produce an ionized gas, and the discharge (16) comprises the electrostatic field (20) and the ionized gas (52).

2. The system of claim 1, wherein the electrostatic tool (200; 400) comprises a gas supply mount (222) configured to mount a gas supply (204) directly to the electrostatic tool (200; 400), wherein the gas passage (234) is configured to connect with the gas supply (204).

3. The system of claim 2, wherein the gas supply mount (222) comprises a gas supply receptacle.

4. The system of claim 1, wherein the electrostatic tool (400) comprises a gas driven turbine (404), an electrical generator (406) coupled to the gas driven turbine (404), and a cascade voltage multiplier (26) coupled to the electrical generator (406).

5. The system of claim 1, wherein the electrostatic tool (200; 400) comprises a liquid passage (240) configured to flow a liquid, the electrostatic tool (200; 400) comprises an atomization system configured to atomize the liquid to produce a liquid spray, the electrostatic applicator (12; 56) is configured to electrically charge the liquid spray to produce a charged liquid spray (72), and the discharge (16) comprises the electrostatic field (20), the ionized gas (52), and the charged liquid spray (72).

6. The system of claim 1, wherein the electrostatic tool (200; 400) comprises a liquid passage (240) configured to supply a liquid, the electrostatic tool (200; 400) comprises an atomization system configured to atomize the liquid to produce a liquid spray, the electrostatic applicator (12; 56) is configured to electrically charge the liquid spray to produce a charged liquid spray (72), and the discharge (16) comprises the electrostatic field and (20) the charged liquid spray (72).

7. The system of claim 6, wherein the electrostatic tool (200; 400) comprises a liquid supply mount configured to mount a liquid supply (402) directly to the electrostatic tool (200; 400), wherein the liquid passage (240) is configured to connect with the liquid supply (402).

8. The system of claim 6, wherein the liquid supply mount comprises a gravity feed container (202).

9. The system of claim 6, wherein the liquid comprises a biocide.

10. The system of claim 1, wherein the electrostatic applicator (12; 56) comprises an electrostatic field diffuser (14).

11. The system of claim 10, wherein the electrostatic field diffuser (14) comprises an electrode grid comprising a plurality of electrodes (208) or an arcuate plate (206).

12. The system of claim 1, wherein the electrostatic tool (200; 400) comprises an electrostatic gun (200).

13. The system of claim 1, wherein the electrostatic tool (200; 400) comprises an electrostatic station (450).

14. The system of claim 13, wherein the electrostatic station (450) comprises a chamber (452), a hand passage (456) into the chamber (452), and a cover (470) configured to open and close the chamber (452).

15. The system of claim 1, further comprising:
an electrostatic module (550) configured to couple with the electrostatic applicator (12; 56), wherein the electrostatic module (550) is configured to transfer an electrostatic charge to a liquid within the electrostatic applicator (12; 56) to create a charged liquid (72; 192), and the electrostatic applicator (12; 56) is configured to spray the charged liquid as a charged spray (72; 192).

16. The system of claim 15, comprising a spray bottle (252), wherein the spray bottle (252) comprises an electrode contact (624) configured to contact an electrode plate (622) of the electrostatic module (550).

17. The system of claim 15, comprising a spray bottle (252), wherein the spray bottle (252) comprises a conductive material portion (664, 666, 670) configured to transfer the electrostatic charge to the liquid within the spray bottle (252), the conductive portion (664) is configured to contact an electrode plate (680) of the electrostatic module (550), and the conductive portion (664, 666, 670) is integrally formed with the spray bottle (252).

18. The system of claim 15, wherein the electrostatic module (550) comprises an electrode (602) configured to puncture the spray bottle (252), and the electrode (602)is configured to transfer the electrostatic charge to the liquid within the spray bottle.

## Patentansprüche

1. System (10; 50; 70; 400), aufweisend:
ein elektrostatisches Werkzeug (200; 400), welches ausgelegt ist, einen Austrag auszugeben, um eine biologische Zelle durch Elektroporation abzutöten, wobei das elektrostatische Werkzeug (200; 400) aufweist:
einen elektrostatischen Applikator (12; 56), welcher ausgelegt ist, ein elektrostatisches Feld (20) auszugeben, wobei der Austrag (16) das elektrostatische Feld (20) aufweist,
**dadurch gekennzeichnet, dass** das elektrostatische Werkzeug (200; 400) einen Gasdurchgang (234) aufweist, welcher ausgelegt ist, ein Gas hindurchzuleiten, dass der elektrostatische Applikator (12; 56) ausgelegt ist, das Gas zu ionisieren, um ein ionisiertes Gas zu erzeugen, und der Austrag (16) das elektrostatische Feld (20) und das ionisierte Gas (52) aufweist.

2. System nach Anspruch 1 wobei das elektrostatische Werkzeug (200; 400) eine Gaszufuhrhalterung (222) aufweist, welche ausgelegt ist, eine Gaszufuhr (204) direkt an dem elektrostatischen Werkzeug (200; 400) zu befestigen, wobei der Gasdurchgang (234) ausgelegt ist, mit der Gaszufuhr (204) in Verbindung zu gelangen.

3. System nach Anspruch 2, wobei die Gaszufuhrhalterung (222) eine Gaszufuhraufnahme aufweist.

4. System nach Anspruch 1, wobei das elektrostatische Werkzeug (400) eine gasbetriebene Turbine (404), einen elektrischen Generator (406), welcher mit der gasbetriebenen Turbine (404) gekoppelt ist, und einen Kaskadenspannungsvervielfacher (26) aufweist, welcher mit dem elektrischen Generator (406) gekoppelt ist.

5. System nach Anspruch 1, wobei das elektrostatische Werkzeug (200; 400) einen Flüssigkeitsdurchgang (240) aufweist, welcher ausgelegt ist, eine Flüssigkeit zu leiten, wobei das elektrostatische Werkzeug (200; 400) ein Zerstäubungssystem aufweist, welches ausgelegt ist, die Flüssigkeit zu zerstäuben, um einen Flüssigkeitssprühstrahl zu erzeugen, wobei der elektrostatische Applikator (12; 56) ausgelegt ist, den Flüssigkeitssprühstrahl elektrisch zu laden, um einen geladenen Flüssigkeitssprühstrahl (72) zu erzeugen, und wobei der Austrag (16) das elektrostatische Feld (20), das ionisierte Gas (52) und den geladenen Flüssigkeitssprühstrahl (72) aufweist.

6. System nach Anspruch 1, wobei das elektrostatische Werkzeug (200; 400) einen Flüssigkeitsdurchgang (240) aufweist, welcher ausgelegt ist, eine Flüssigkeit zuzuführen, wobei das elektrostatische Werkzeug (200; 400) ein Zerstäubungssystem aufweist, welches ausgelegt ist, die Flüssigkeit zu zerstäuben, um einen Flüssigkeitssprühstrahl zu erzeugen, wobei der elektrostatische Applikator (12; 56) ausgelegt ist, den Flüssigkeitssprühstrahl elektrisch zu laden, um einen geladenen Flüssigkeitssprühstrahl (72) zu erzeugen, und wobei der Austrag (16) das elektrostatische Feld (20) und den geladenen Flüssigkeitssprühstrahl (72) aufweist.

7. System nach Anspruch 6, wobei das elektrostatische Werkzeug (200; 400) eine Flüssigkeitszufuhrhalterung aufweist, welche ausgelegt ist, eine Flüssigkeitszufuhr (402) direkt an dem elektrostatischen Werkzeug zu montieren, wobei der Flüssigkeitsdurchgang (240) ausgelegt ist, mit der Flüssigkeitszufuhr (402) in Verbindung zu gelangen.

8. System nach Anspruch 6, wobei die Flüssigkeitszufuhrhalterung einen Behälter (202) mit Schwerkraftzufuhr aufweist.

9. System nach Anspruch 6, wobei die Flüssigkeit ein Biozid aufweist.

10. System nach Anspruch 1, wobei der elektrostatische Applikator (12; 56) einen elektrostatischen Felddiffusor (14) aufweist.

11. System nach Anspruch 10, wobei der elektrostatische Felddiffusor (14) ein Elektrodengitter mit einer Vielzahl von Elektroden (208) oder eine gebogene Platte (206) aufweist.

12. System nach Anspruch 1, wobei das elektrostatische Werkzeug (200; 400) eine elektrostatische Pistole (200) aufweist.

13. System nach Anspruch 1, wobei das elektrostatische Werkzeug (200; 400) eine elektrostatische Station (450) aufweist.

14. System nach Anspruch 13, wobei die elektrostatische Station (450) eine Kammer (452), einen Handdurchgang (456) in die Kammer (452) und eine Abdeckung (470) aufweist, welche ausgelegt ist, die Kammer (452) zu öffnen und zu verschließen.

15. System nach Anspruch 1, ferner aufweisend:
ein elektrostatisches Modul (550), welches ausgelegt ist, mit dem elektrostatischen Applikator (12; 56) gekoppelt zu werden, wobei das elektrostatische Modul (550) ausgelegt ist, eine elektrostatische Ladung auf eine Flüssigkeit in dem elektrostatischen Applikator (12; 56) zu übertragen, um eine geladene Flüssigkeit (72; 192) zu erzeugen, und wobei der elektrostatische Applikator (12; 56) ausgelegt ist, die geladene Flüssigkeit als einen geladenen Sprühstrahl (72; 192) zu versprühen.

16. System nach Anspruch 15, eine Sprühflasche (252) aufweisend, wobei die Sprühflasche (252) einen Elektrodenkontakt (624) aufweist, welcher ausgelegt ist, eine Elektrodenplatte (622) des elektrostatischen Moduls (550) zu kontaktieren.

17. System nach Anspruch 15, aufweisend eine Sprühflasche (252), wobei die Sprühflasche (252) einen leitfähigen Materialteil (664, 666, 670) aufweist, welcher ausgelegt ist, die elektrostatische Ladung auf die Flüssigkeit in der Sprühflasche (252) zu übertragen, wobei der leitfähige Teil (664) ausgelegt ist, eine Elektrodenplatte (680) des elektrostatischen Moduls (550) zu kontaktieren, und wobei der leitfähige Teil (664, 666, 670) integral mit der Sprühflasche (252) ausgebildet ist.

18. System nach Anspruch 15, wobei das elektrostatische Modul (550) eine Elektrode (602) aufweist, welche ausgelegt ist, die Sprühflasche (252) zu durchstechen, und wobei die Elektrode (602) ausgelegt ist, die elektrostatische Ladung auf die Flüssigkeit in der Sprühflasche zu übertragen.

## Revendications

1. Système (10 ; 50 ; 70 ; 400), comprenant :
un outil électrostatique (200 ; 400) configuré pour délivrer une décharge pour tuer une cellule biologique par électroporation, dans lequel l'outil électrostatique (200 ; 400) comprend :
un applicateur électrostatique (12 ; 56) configuré pour délivrer un champ électrostatique (20), dans lequel la décharge (16) comprend le champ électrostatique (20),
**caractérisé en ce que** l'outil électrostatique (200 ; 400) comprend un passage à gaz (234) configuré pour l'écoulement d'un gaz, l'applicateur électrostatique (12 ; 56) est configuré pour ioniser le gaz afin de produire un gaz ionisé, et la décharge (16) comprend le champ électrostatique (20) et le gaz ionisé (52).

2. Système selon la revendication 1, dans lequel l'outil électrostatique (200 ; 400) comprend une monture d'alimentation de gaz (220) configurée pour monter une alimentation de gaz (204) directement sur l'outil électrostatique (200 ; 400), dans lequel le passage à gaz (234) est configuré pour être connecté avec l'alimentation de gaz (204).

3. Système selon la revendication 2, dans lequel la monture d'alimentation de gaz (222) comprend un réceptacle d'alimentation de gaz.

4. Système selon la revendication 1, dans lequel l'outil électrostatique (400) comprend une turbine entraînée par un gaz (404), un générateur électrique (406) couplé à la turbine (404) entraînée par un gaz, et un multiplicateur de voltage en cascade (26) couplé au générateur électrique (406).

5. Système selon la revendication 1, dans lequel l'outil électrostatique (200 ; 400) comprend un passage à liquide (240) configuré pour l'écoulement d'un liquide, l'outil électrostatique (200 ; 400) comprend un système d'atomisation configuré pour atomiser le liquide afin de produire une pulvérisation de liquide, l'applicateur électrostatique (12 ; 56) est configuré pour charger électriquement la pulvérisation de liquide et produire une pulvérisation de liquide chargé (72), et la décharge (16) comprend le champ électrostatique (20), le gaz ionisé (52), et la pulvérisation de liquide chargé (72).

6. Système selon la revendication 1, dans lequel l'outil électrostatique (200 ; 400) comprend un passage à liquide (240) configuré pour l'alimentation d'un liquide, l'outil électrostatique (200 ; 400) comprend un système d'atomisation configuré pour atomiser le liquide et produire une pulvérisation de liquide, l'applicateur électrostatique (12 ; 56) est configuré pour charger électriquement la pulvérisation de liquide pour produire une pulvérisation de liquide chargé (72), et la décharge (16) comprend le champ électrostatique (20) et la pulvérisation de liquide chargé (72).

7. Système selon la revendication 6, dans lequel l'outil électrostatique (200 ; 400) comprend une monture d'alimentation de liquide configurée pour monter une alimentation de liquide (402) directement sur l'outil électrostatique (200 ; 400), dans lequel le passage à liquide (240) est configuré pour être connecté avec l'alimentation de liquide (402).

8. Système selon la revendication 6, dans lequel la monture d'alimentation de liquide comprend un conteneur d'alimentation par gravité (202).

9. Système selon la revendication 6, dans lequel le liquide comprend un produit biocide.

10. Système selon la revendication 1, dans lequel l'applicateur électrostatique (12 ; 56) comprend un diffuseur de champ électrostatique (14).

11. Système selon la revendication 10, dans lequel le diffuseur de champ électrostatique (14) comprend une grille électrode comprenant une pluralité d'électrodes (208) ou une plaque arquée (206).

12. Système selon la revendication 1, dans lequel l'outil électrostatique (200 ; 400) comprend un canon électrostatique (200).

13. Système selon la revendication 1, dans lequel l'outil électrostatique (200 ; 400) comprend une station électrostatique (450).

14. Système selon la revendication 13, dans lequel la station électrostatique (450) comprend une chambre (452), un passage à main (456) jusque dans la chambre (452), et un couvercle (470) configuré pour ouvrir et fermer la chambre (452).

15. Système selon la revendication 1, comprenant en outre :
un module électrostatique (550) configuré pour être couplé avec l'applicateur électrostatique (12 ; 56), dans lequel le module électrostatique (550) est configuré pour transférer une charge électrostatique à un liquide à l'intérieur de l'applicateur électrostatique (12 ; 56) afin de créer un liquide chargé (72 ; 192), et l'applicateur électrostatique (12 ; 56) est configuré pour pulvériser le liquide chargé sous forme d'une pulvérisation chargée (72 ; 192).

16. Système selon la revendication 15, comprenant une bouteille de pulvérisation (252), dans lequel la bouteille de pulvérisation (252) comprend un contact pour électrode (624) configuré pour venir en contact avec une plaque électrode (622) du module électrostatique (550).

17. Système selon la revendication 15, comprenant une bouteille de pulvérisation (252), dans lequel la bouteille de pulvérisation (252) comprend une portion en matériau conducteur (664, 666, 670) configurée pour transférer la charge électrique au liquide à l'intérieur de la bouteille de pulvérisation (252), la portion conductrice (664) est configurée pour venir en contact avec une plaque électrode (680) du module électrostatique (550), et la portion conductrice (664, 666, 670) est formée intégralement avec la bouteille de pulvérisation (252).

18. Système selon la revendication 15, dans lequel le module électrostatique (550) comprend une électrode (602) configurée pour perforer la bouteille de pulvérisation (252), et l'électrode (602) est configuré pour transférer la charge électrostatique au liquide à l'intérieur de la bouteille de pulvérisation.
